# EUROPEAN PATENT APPLICATION

(11) **EP 2 308 490 A1**
(43) Date of publication of application: **13.04.2011**
(21) Application number: 10193364.6
(22) Date of filing: 29.12.2006
(51) Int. Cl.: A61K 31/426, A61K 31/47, A61P 31/18

(54) **Methods for improving the pharmacokinetics of hiv integrase inhibitors**

(30) Priority: 30.12.2005 US 755039 P; 06.01.2006 US 756631 P; 01.02.2006 US 763901 P
(62) Divisional of application: 06848393.2
(71) Applicant: Gilead Sciences, Inc., Foster City, CA 94404 (US); Japan Tobacco, Inc., Tokyo 105-8422 (JP)
(72) Inventor: Kearney, Brian P., San Francisco, CA 94110 (US); Kakee, Atsuyuki, Tokyo 105-8422 (JP); Kawaguchi, Isao, Tokyo 105-8422 (JP)
(74) Representative: Reitstötter - Kinzebach

(57) **Abstract**

The invention provides methods for improving the pharmacokinetics of an HIV integrase inhibiting compound by administering food and/or ritonavir or a pharmaceutically acceptable salt thereof with the HIV integrase inhibitor.

## Description

### PRIORITY OF INVENTION

This application claims priority to United States Provisional Patent Applications 60/755,039, filed 30 December 2005; 60/756,631, filed 06 January 2006; and 60/763,901, filed 01 February 2006.

### BACKGROUND OF THE INVENTION

Infection by the retrovirus known as human immunodeficiency virus (HIV) continues to be a serious human health problem. Methods for treating HIV infections include administering agents that inhibit the activity of viral enzymes that are essential to the life cycle of the virus.

Ritonavir ((2S,3S,5S)-5-(N-(N-((N-Methyl-N-((2-isopropyl-4-thiazolyl)methyl)amino)-carbonyl)-L-valinyl)amino)-2-(N-((5-thiazolyl)methoxycarbonyl)amino)-1,6-diphenyl-3-hydroxyhexane) is an HIV protease inhibitor that can be synthesized by procedures disclosed in International Patent Application Publication Number WO 1994/14436 and United States Patent Number 5567823. As a protease inhibitor, ritonavir can be effective in humans for inhibiting an HIV infection. Ritonavir has also been shown as an inhibitor of the metabolic enzyme cytochrome P450 monooxygenase, particularly, the 3A4 isoform (CYP 3A4) involved in the metabolic pathway of many drugs. *See* U.S. Pat. Nos. 5541206, 5635523, 5648497, 5674882,5846987 and 5886036.

Protease inhibitors are metabolized by cytochrome P450 monooxygenase, leading to unfavorable pharmacokinetics and the need for more frequent and higher doses than are desirable. Administration of such drugs with an agent that inhibits metabolism by cytochrome P450 monooxygenase can improve the pharmacokinetics (*e.g*., increases in half-life, time to peak plasma concentration and blood levels) of the drug.

Ritonavir can be used to improve the pharmacokinetics of certain HIV protease inhibitors that are metabolized by cytochrome P450 monooxygenase. See United States Patent Numbers 6,037,157 and 6,703,403. Co-administration of ritonavir with a drug metabolized by cytochrome P450 monooxygenase, especially, the P450 3A4 isoform (isozyme), may cause an improvement in the pharmacokinetics of such a drug. More particularly, co-administration of ritonavir with another HIV protease inhibitor that is metabolized by cytochrome P450 monooxygenase may result in an improvement in the pharmacokinetics of the HIV protease inhibitor. In this type of combination therapy, ritonavir may be used at subtherapeutic dosages, that is, dosages less than that used to meaningfully suppress viral replication, yet high enough to inhibit the cytochrome P450 monooxygenase and boost the pharmacokinetics of the other HIV protease inhibitor.

A series of 4-oxoquinolines including the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid have been identified as anti-human immunodeficiency virus (HIV) agents. *See* United States Patent Application Serial Number 10/492,833, filed November 20, 2003, which was published as United States Patent Application Publication Number 2005/0239819. Specifically, 6-(3-chloro-2-fluorobenzyl)-1-[(*2S*)-1-hydroxy-3-methylbutan-2-yl]-7-methoxy-4-oxo-1,4-dihydroquinoline-3-carboxylic acid has been described as having inhibitory activity against the integrase protein of HIV. *Id.* HIV belongs to the retrovirus family and is a causative agent of the acquired immunodeficiency syndrome (AIDS). Accordingly, a pharmaceutical agent that reduces the virus load, viral genome, or replication of HIV in the body, may be effective for the treatment or prophylaxis of AIDS.

Currently, there is a need for agents and methods that are useful for increasing the bioavailability or absorption of integrase inhibitors such as 6-(3-chloro-2-fluorobenzyl)-1-[(*2S*)-1-hydroxy-3-methylbutan-2-yl]-7-methoxy-4-oxo-1,4-dihydroquinoline-3-carboxylic acid in order to increase their therapeutic effect in a patient. In particular, there is a need for agents and methods that improve the pharmacokinetics of such an integrase inhibitor so that an acceptable therapeutic effect can be achieved by once daily administration. There is also generally a need to improve the pharmacokinetics of drugs (e.g. integrase inhibitors), that are useful for treating HIV infection.

### SUMMARY OF THE INVENTION

The invention relates to a method of improving the pharmacokinetics of 4-oxoquinoline compounds. The invention further relates to a method of inhibiting retroviral integrases, particularly, of inhibiting human immunodeficiency virus (HIV) integrase, and a method of inhibiting a retroviral infection, particularly, an HIV infection.

In one embodiment the invention provides agents and methods that are useful for increasing the bioavailability or absorption of integrase inhibitors such as 6-(3-chloro-2-fluorobenzyl)-1-[(*2S*)-1-hydroxy-3-methylbutan-2-yl]-7-methoxy-4-oxo-1,4-dihydroquinoline-3-carboxylic acid 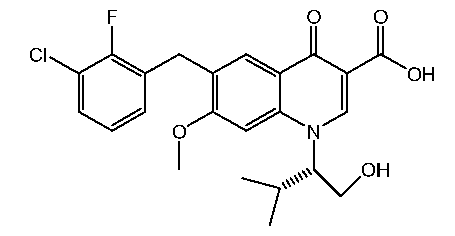 (Compound **1**) in order to increase their therapeutic effect in a patient.

In one embodiment, the invention provides a method for improving the pharmacokinetics of an integrase inhibitor such as Compound **1**, by administering the integrase inhibitor to a patient with ritonavir or a pharmaceutically acceptable salt thereof.

In one embodiment the invention provides a method of improving the pharmacokinetics of a HIV integrase inhibitor, comprising administering to a patient in need of the inhibitor an effective boosting amount of ritonavir or a pharmaceutically acceptable salt thereof, such that the inhibitor possesses a more efficacious pharmacokinetic profile than it would without the addition of ritonavir.

In another embodiment the invention provides a method for improving the pharmacokinetics of a 4-oxoquinoline compound according to formula (I): 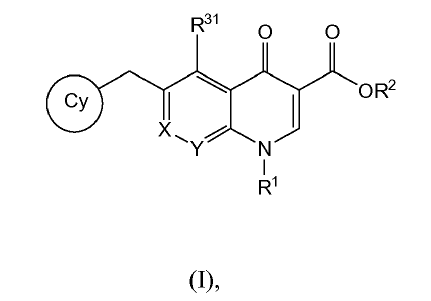 where,
ring Cy is a C₃₋₁₀ carbon ring group or a heterocyclic group, each group being optionally substituted by 1 to 5 substituents selected from group A;
the heterocyclic group is a saturated or unsaturated ring comprising at least one heteroatom selected from the group consisting of nitrogen, oxygen and sulfur;
group A is cyano, phenyl, nitro, halogen, C₁₋₄ alkyl, halo C₁₋₄ alkyl, halo C₁₋₄ alkyloxy, -OR^{a1}, -SR^{a1}, -NR^{a1}R^{a2}, -CONR^{a1}R^{a2}, -SO₂NR^{a1}R^{a2}, - COR^{a3}, -NR^{a1}COR^{a3}, -SO₂R^{a3}, -NR^{a1}SO₂R^{a3}, -COOR^{a1} or -NR^{a2} COOR^{a3};
R^{a1} and R^{a2} are the same or different and each is H, C₁₋₄ alkyl or benzyl;
R^{a3} is C₁₋₄ alkyl;
R¹ is selected from group B or is C₁₋₁₀ alkyl optionally substituted by 1 to 3 substituents selected from halogen or group B;
group B is:
a C₃₋₁₀ carbon ring optionally substituted by 1 to 5 substituents selected from group A,
a heterocyclic group optionally substituted by 1 to 5 substituents selected from group A,
   - OR^{a4},
   - SR^{a4},
   - NR^{a4}R^{a5},
   - CONR^{a4}R^{a5},
   - SO₂NR^{a4}R^{a5},
   - COR^{a6},
   - NR^{a4}COR^{a6},
   - SO₂R^{a6},
   - NR^{a4}SO₂R^{a6},
   - COOR^{a4} or
   - NR^{a5} COOR^{a6};
R^{a4} and R^{a5} are the same or different and each is:
H,
C₁₋₄ alkyl,
a C₃₋₁₀ carbon ring group optionally substituted by 1 to 5 substituents selected from group A or
a heterocyclic group optionally substituted by 1 to 5 substituents selected from the group A;
R^{a6} is
C₁₋₄ alkyl,
a C₃₋₁₀ carbon ring group optionally substituted by 1 to 5 substituents selected from group A or
a heterocyclic group optionally substituted by 1 to 5 substituents selected from group A;
R² is H or C₁₋₄ alkyl;
R³¹ is H, cyano, hydroxy, amino, nitro, halogen, C₁₋₄ alkyl, C₁₋₄ alkoxy, C₁₋₄ alkylsulfanyl, halo C₁₋₄ alkyl or halo C₁₋₄ alkyloxy group;
X is C-R³² or N;
Y is C-R³³ or N;
R³² and R³³ are the same or different and each is:
H,
cyano,
nitro,
halogen,
a C₃₋₁₀ carbon ring group optionally substituted by 1 to 5 substituents selected from group A,
a heterocyclic group optionally substituted by 1 to 5 substituents selected from group A, C₁₋₁₀ alkyl optionally substituted by 1 to 3 substituents selected from halogen or group B,
   - OR^{a7},
   - SR^{a7},
   - NR^{a7}R^{a8},
   - NR^{a7}COR^{a9},
   - COOR^{a10} or
   - N=CH-NR^{a10}R^{a11};
R^{a7} and R^{a8} are the same or different and each is selected from H, group B or C₁₋₁₀ alkyl optionally substituted by 1 to 3 substituents selected from halogen or group B;
R^{a9} is C₁₋₄ alkyl; and
R^{a10} and R^{a11} are the same or different and each is H or C₁₋₄ alkyl
comprising administering the compound of formula (I) or a pharmaceutically acceptable salt thereof, and ritonavir or a pharmaceutically acceptable salt thereof, to a patient in need thereof.

In one embodiment the invention provides a method of improving the pharmacokinetics of a compound of Formula (I) comprising administering to a patient in need of the compound or a pharmaceutically acceptable salt thereof an effective amount of ritonavir or a pharmaceutically acceptable salt thereof.

In one embodiment the invention provides a method for increasing the blood level of a compound of Formula (I) in a patient being treated with the compound or a pharmaceutically acceptable salt thereof, comprising administering to a patient in need of the compound or a pharmaceutically acceptable salt thereof an effective amount of ritonavir or a pharmaceutically acceptable salt thereof.

In one embodiment the invention provides a method of inhibiting HIV integrase in a patient in need of such treatment comprising administering a compound of Formula (I) or a pharmaceutically acceptable salt thereof and an effective amount of ritonavir or a pharmaceutically acceptable salt thereof.

In one embodiment the invention provides a method of increasing the bioavailability of compound **1** in a patient. The method comprises administering to the patient a therapeutically effective amount of compound **1** with food. The increase in bioavailability of the compound may be observed by an increase in the maximum plasma concentration or by an increase in the area under the plasma concentration time curve (AUC) compared to that if the compound was administered without food.

In one embodiment the invention provides a method of increasing the absorption of compound **1** in a patient, comprising administering to the patient a therapeutically effective amount of compound **1** with food. Absorption of the compound may be measured by the concentration attained in the bloodstream after administration of the compound. An increase in absorption may be observed by an increase in the maximum plasma concentration or by an increase in the area under the plasma concentration time curve (AUC) compared to that if the compound was administered without food.

In one embodiment the invention provides a method for inhibiting activity of a retrovirus integrase in a patient, comprising administering to the patient a therapeutically effective amount of compound **1** with food.

In one embodiment the invention provides a method for the treatment or prophylaxis of a retrovirus infection in a patient, comprising administering to the patient a therapeutically effective amount of compound **1** with food.

In one embodiment the invention provides a kit comprising: (1) a pharmaceutical composition comprising compound **1,** or a pharmaceutically acceptable salt thereof, and a pharmaceutically acceptable carrier; (2) prescribing information; and (3) a container. The prescribing information includes advice caution or instruction to a patient relating to administering compound **1** with food.

In another embodiment the invention provides a kit comprising an integrase inhibitor such as Compound **1,** prescribing information, and a container, wherein the prescribing information includes information regarding administration of the compound to improve its bioavailability.

In one embodiment, the invention provides a method for improving the pharmacokinetics of an integrase inhibitor such as compound **1,** by administering the integrase inhibitor to a patient with ritonavir or a pharmaceutically acceptable salt thereof and with food.

In one embodiment the invention provides a method of increasing the bioavailability of compound **1** in a patient comprising administering to the patient a therapeutically effective amount of compound **1** with ritonavir and with food.

In one embodiment the invention provides a method of increasing the absorption of compound **1** in a patient, comprising administering to the patient a therapeutically effective amount of compound **1** with ritonavir and with food.

In one embodiment the invention provides a method for inhibiting activity of a retrovirus integrase in a patient, comprising administering to the patient a therapeutically effective amount of compound **1** with ritonavir and with food.

In one embodiment the invention provides a method for the treatment or prophylaxis of a retrovirus infection in a patient, comprising administering to the patient a therapeutically effective amount of compound **1** with ritonavir and with food.

In one embodiment the invention provides the use of ritonavir or a pharmaceutically acceptable salt thereof for the manufacture of a medicament for improving the pharmacokinetics of an HIV integrase inhibitor (e.g. a compound of Formula (I)) or a pharmaceutically acceptable salt thereof in a patient.

In one embodiment the invention provides the use of ritonavir or a pharmaceutically acceptable salt thereof, and a compound of Formula (I) or a pharmaceutically acceptable salt thereof, for the manufacture of a medicament for inhibiting HIV integrase in a patient.

In one embodiment the invention provides the use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or its pharmaceutically acceptable salt for the manufacture of a medicament for increasing the bioavailability of the compound comprising administering to a patient a therapeutically effective amount of the compound or a pharmaceutically acceptable salt thereof to be administered with food.

In one embodiment the invention provides the use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or its pharmaceutically acceptable salt for the manufacture of a medicament for increasing the absorption of the compound in a patient, comprising administering to the patient a therapeutically effective amount of the compound or a pharmaceutically acceptable salt thereof to be administered with food.

In one embodiment the invention provides use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or its pharmaceutically acceptable salt for the manufacture of a medicament for inhibiting activity of a retrovirus integrase in a patient, comprising administering to the patient a therapeutically effective amount of the compound or a pharmaceutically acceptable salt thereof to be administered with food.

In one embodiment the invention provides the use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or its pharmaceutically acceptable salt for the manufacture of a medicament for the treatment or prophylaxis of a retrovirus infection in a patient, comprising administering to the patient a therapeutically effective amount of the compound or a pharmaceutically acceptable salt thereof to be administered with food.

In one embodiment the invention provides a kit comprising: (1) a pharmaceutical composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, and a pharmaceutically acceptable carrier; (2) prescribing information; and (3) a container; wherein the prescribing information includes advice regarding administering 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof with food. In one embodiment the kit can optionally further comprise ritonavir or a pharmaceutically acceptable salt thereof.

In one embodiment the invention provides use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for the manufacture of a medicament for increasing the bioavailability of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid in a patient when the medicament is administered with food, to be administered with ritonavir or a pharmaceutically acceptable salt thereof.

In one embodiment the invention provides use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for the manufacture of a medicament for increasing the absorption of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid in a patient when the medicament is administered with food, to be administered with ritonavir or a pharmaceutically acceptable salt thereof.

In one embodiment the invention provides use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for the manufacture of a medicament for inhibiting activity of a retrovirus integrase in a patient when the medicament is administered with food, to be administered with ritonavir or a pharmaceutically acceptable salt thereof.

In one embodiment the invention provides use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for the manufacture of a medicament for the treatment or prophylaxis of a retrovirus infection in a patient when the medicament is administered with food, to be administered with ritonavir or a pharmaceutically acceptable salt thereof.

In one embodiment the invention provides a pharmaceutical composition comprising ritonavir or a pharmaceutically acceptable salt thereof for improving the pharmacokinetics of an HIV integrase inhibitor in a patient.

In one embodiment the invention provides a pharmaceutical composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for increasing the bioavailability of the compound to be administered with food.

In one embodiment the invention provides a pharmaceutical composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for increasing the absorption of the compound in a patient to be administered with food.

In one embodiment the invention provides a pharmaceutical composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for inhibiting activity of a retrovirus integrase in a patient to be administered with food.

In one embodiment the invention provides a pharmaceutical composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for the treatment or prophylaxis of a retrovirus infection in a patient to be administered with food.

In one embodiment the invention provides an anti-retroviral agent comprising ritonavir or a pharmaceutically acceptable salt thereof for improving the pharmacokinetics of an HIV integrase inhibitor in a patient.

In one embodiment the invention provides an anti-retroviral agent composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for increasing the bioavailability of the compound to be administered with food.

In one embodiment the invention provides an anti-retroviral agent comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for increasing the absorption of the compound in a patient to be administered with food.

In one embodiment the invention provides an anti-retroviral agent comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for inhibiting activity of a retrovirus integrase in a patient to be administered with food.

In one embodiment the invention provides an anti-retroviral agent comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for the treatment or prophylaxis of a retrovirus infection in a patient to be administered with food.

In one embodiment the invention provides the use of an integrase inhibitor or a pharmaceutically acceptable salt thereof for the manufacture of a medicament for oral administration with food, for achieving enhansed bioavailibility of the integrase inhibitor or the pharmaceutically acceptable salt thereof in the treatment of an integrase responsive condition (e.g. a retrovirus infection such as an HIV infection or AIDS).

In one embodiment the invention provides the use of an integrase inhibitor (e.g. a compound of Formula (I) such as Compound **1**) or a pharmaceutically acceptable salt thereof for the manufacture of a medicament for oral administration with food, for achieving increased absorption of the integrase inhibitor or the pharmaceutically acceptable salt thereof in the treatment of an integrase responsive condition (e.g. a retrovirus infection such as an HIV infection or AIDS).

In one embodiment the invention provides the use of an integrase inhibitor (e.g. a compound of Formula (I) such as Compound **1**) or a pharmaceutically acceptable salt thereof for the manufacture of a medicament for administration with ritonavir or a pharmaceutically acceptable salt thereof for the treatment of an integrase responsive condition (e.g. a retrovirus infection such as an HIV infection or AIDS).

In one embodiment the invention provides the use of ritonavir or a pharmaceutically acceptable salt thereof for the manufacture of a medicament for administration with an integrase inhibitor (e.g. a compound of Formula (I) such as Compound **1**) or a pharmaceutically acceptable salt thereof for the treatment of an integrase responsive condition (e.g. a retrovirus infection such as an HIV infection or AIDS).

In one embodiment the invention provides the use of an integrase inhibitor (e.g. a compound of Formula (I) such as Compound **1**) or a pharmaceutically acceptable salt thereof, and ritonavir or a pharmaceutically acceptable salt thereof, for the manufacture of a medicament for the treatment of an integrase responsive condition (e.g. a retrovirus infection such as an HIV infection or AIDS).

In one embodiment the invention provides the use of ritonavir or a pharmaceutically acceptable salt thereof for the manufacture of a medicament for administration with an integrase inhibitor (e.g. a compound of Formula (I) such as Compound **1**) or a pharmaceutically acceptable salt thereof for the treatment of an integrase responsive condition (e.g. a retrovirus infection such as an HIV infection or AIDS).

In one embodiment the invention provides the use of an integrase inhibitor (e.g. a compound of Formula (I) such as Compound **1**) or a pharmaceutically acceptable salt thereof for the manufacture of a medicament for administration with ritonavir or a pharmaceutically acceptable salt thereof and for administration with food, for the treatment of an integrase responsive condition (e.g. a retrovirus infection such as an HIV infection or AIDS).

In one embodiment the invention provides the use of ritonavir or a pharmaceutically acceptable salt thereof for the manufacture of a medicament for administration with an integrase inhibitor (e.g. a compound of Formula (I) such as Compound **1**) or a pharmaceutically acceptable salt thereof and for administration with food, for the treatment of an integrase responsive condition (e.g. a retrovirus infection such as an HIV infection or AIDS).

These and other advantages of the invention, as well as additional inventive features, will be apparent from the description of the invention provided herein.

### BRIEF DESCRIPTION OF THE DRAWING

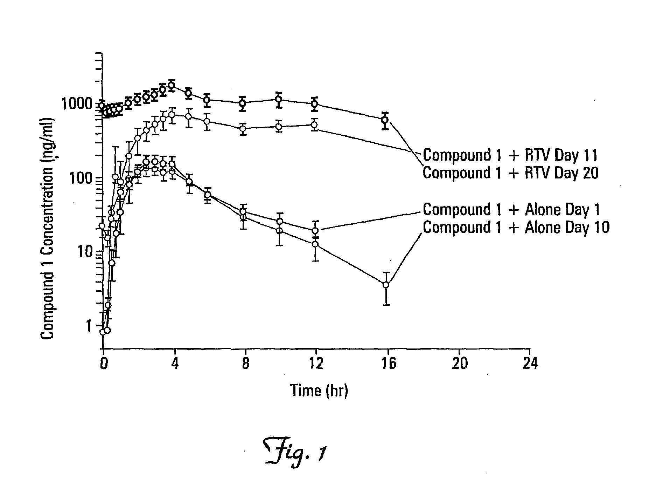

FIG. 1 is graph of plasma concentration versus time for Compound **1** alone and in combination with ritonavir.
FIG. 2 is a graph plotted on a linear scale of the plasma concentration of 6-(3-chloro-2-fluorobenzyl)-1-[(2S)-1-hydroxy-3-methylbutan-2-yl]-7-methoxy-4-oxo-1,4-dihydroquinoline-3-carboxylic acid (Compound **1**) after administration thereof in the fasted and fed states.
FIG. 3 illustrates data from Example 3.
FIG. 4 illustrates data from Example 3.
FIG. 5 illustrates data from Example 3.

### DETAILED DESCRIPTION OF THE INVENTION

### Ritonavir Effects

Compounds of the formula (I) are HIV integrase inhibitors. A specific group of compounds of formula (I) are compounds of formula (II): 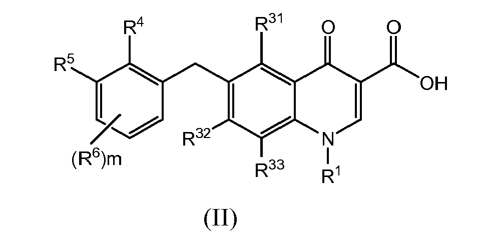 wherein,
R⁴ and R⁶ are the same or different and each is selected from group A;
R⁵ is selected from H or group A;
or R⁴ and R⁵ together form a ring that fuses to the benzene ring to which
they are bonded;
m is 0 to 3; and
R¹, R³¹, R³² and R³³ are each defined the same as in formula (I);
provided that when m is 2 or 3, then R⁶ of each m is optionally the same or different.

A preferred compound of formula (I) is Compound 1:

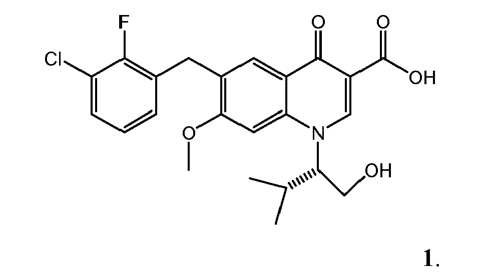

Compounds of formula (I) are described in United States Patent Application Publication Number US 2005/0239819, which is hereby incorporated herein in its entirety. Compound **1** can be found on page 76, at Example 4-32, of this document. Methods of preparation and use for this and other compounds of formulas (I) and (II) are also described in this document.

In accordance with one embodiment of the invention, there is disclosed a method of improving the pharmacokinetics of a drug (or a pharmaceutically acceptable salt thereof) by administering ritonavir or a pharmaceutically acceptable salt thereof with the drug. The drug is preferably an HIV integrase inhibitor. Furthermore, the drug is preferably metabolized by cytochrome P450 monooxygenase. When administered, the two agents can be formulated as separate compositions that are administered at the same or different times (*e.g*., concurrently, consecutively or sequentially), or they can be formulated and administered as a single composition.

Certain HIV protease inhibitors that are metabolized by cytochrome P450 monooxygenase and that would benefit from administration with ritonavir are described in United States Patent Numbers 6,037,157 and 6,703,403, which are hereby incorporated herein by reference in their entirety. These patents describe formulating and dosing regimens that can be used with ritonavir.

Formulating and dosing regimens for HIV integrase inhibitors may be found in United States Patent Application Publication Number US 2004/0167124, which is hereby incorporated herein by reference in its entirety. The aforementioned regimens may be applied to the invention described herein. In the case of combination therapy, for example, about 20mg to about 500mg of a compound of formula (I) or (II) such as Compound **1** may be administered with about 10 mg to about 1200mg of ritonavir per day. In one specific embodiment of the invention about 20mg to about 500mg of a compound of formula (I) or (II) such as Compound **1** may be administered with about 10 mg to about 600mg of ritonavir per day. In an embodiment of the invention, a suitable dose of compound 1 is about 20 mg, 50 mg, 75 mg, 85 mg, 100 mg, 125 mg, 150 mg, 175 mg or 200mg, more specifically about 85 mg, about 125 mg or about 150 mg. In an embodiment of the invention, a suitable dose of ritonavir is between about 20 mg to about 200 mg, specifically between about 50 mg to about 125mg, more specifically about 100 mg. However, higher and lower dosages of each agent may be efficacious.

In one embodiment of daily administration, preferable amounts of Compound 1 and ritonavir are dosages which can achieve blood concentration of Compound 1 maintaining over IC₉₅ (f.e. protein binding-adjusted in vitro IC₉₅ 100nM) during 24 hours.

In one embodiment of daily administration, preferable amounts of Compound 1 and ritonavir are dosages which can achieve blood concentration of Compound 1 maintaining over EC₉₀ (f.e. approximately 170 ng/ml in Emax model) during 24 hours.

In one embodiment, preferable amounts of the compound 1 is dosages given to patients which can be achieved that mean reductions in HIV RNA as anti-HIV activity is more than 1.5 log₁₀ copies/ml, preferably 2.0 log₁₀.

In a specific embodiment the invention provides a method for improving the pharmacokinetics of an HIV integrase inhibitor (or a pharmaceutically acceptable salt thereof), particularly, one that is metabolized by cytochrome P450 monooxygenase, more particularly, the CYP 3A4 isoform, in a patient in need of such treatment, by administering or coadministering ritonavir or a pharmaceutically acceptable salt thereof with the integrase inhibitor. Such a combination of ritonavir or a pharmaceutically acceptable salt thereof and an HIV integrase inhibitor or a pharmaceutically acceptable salt thereof that is metabolized by cytochrome P450 monooxygenase is useful for inhibiting HIV integrase in a patient and is also useful for inhibition, treatment or prophylaxis of an HIV infection or AIDS (acquired immune deficiency syndrome) in a patient.

Patients include any living beings, particularly, mammals (*e.g*., humans).

One aspect of the invention provides the use of an effective amount of ritonavir to boost the pharmacokinetics of an HIV integrase inhibitor. An effective amount of ritonavir, that is, the amount required to boost the HIV integrase inhibitor, is the amount necessary to improve the pharmacokinetic profile of the HIV integrase inhibitor when compared to its profile when used alone. The inhibitor possesses a better efficacious pharmacokinetic profile than it would without the addition of ritonavir. The amount of ritonavir used to boost an integrase inhibitor can be subtherapeutic (*e.g*., dosages below the amount of ritonavir conventionally used for therapeutically treating HIV infection in a patient). A boosting dose of ritonavir is subtherapeutic for treating HIV infection, yet high enough to effect modulation of the metabolism of the compounds of formulas (I) and (II), such that their exposure in a patient is boosted by increased bioavailability, increased blood levels, increased half life, increased time to peak plasma concentration, increased/faster inhibition of HIV integrase and/or reduced systematic clearance.

Compound **1** is a HIV integrase inhibitor that is metabolized by cytochrome P450 monooxygenase, particularly, the CYP 3A isoform. It has now been found that ritonavir can be used to boost the pharamacokinetics of compounds of Fomula (I) as well as other HIV integrase inhibitors. Ritonavir is particularly useful for boosting the effects of integrase inhibitors that are metabolized by cytochrome P450 monooxygenase (e.g. the CYP 3A isoform). The degree of such boosting was unexpectedly substantial. Ritonavir can limit the first-pass effect of these compounds. Ritonavir may also limit the secondary-pass (systemic or hepatic metabolism/clearance) effects of these compounds.

According to the methods of the invention the integrase inhibitor (or the compound of Formula (I)) or the pharmaceutically acceptable salt thereof can also be administered with one or more other agents that are useful for treating viral infections, such as, stavudine, emtricitabine, tenofovir, emtricitabine, abacavir, lamivudine, zidovudine, didanosine, zalcitabine, phosphazide, efavirenz, nevirapine, delavirdine, tipranavir, saquinavir, indinavir, atazanavir, nelfinavir, amprenavir, samprenavir, fosamprenavir, lopinavir, ritonavir, enfuvirtide, Fozivudine tidoxil, Alovudine, Dexelvucitabine, Apricitabine, Amdoxovir, Elvucitabine (ACH126443), Racivir (racemic FTC, PSI-5004), MIV-210, KP-1461, fosalvudine tidoxil (HDP 99.0003), AVX756, Dioxolane Thymine (DOT), TMC-254072, INK-20, 4'-Ed4T, TMC-125 (etravirine), Capravirine, TMC-278 (rilpivirine), GW-695634, Calanolide A, BILR 355 BS, and VRX 840773, or pharmaceutically acceptable salts thereof. Although the above list includes tradenames for several compounds, it is to be understood that the reference to the tradename also includes the underlying active chemical agent, regardless of source. The kits of the invention can also optionally further comprise one or more other agents selected from the above list.

In one embodiment of the invention, the methods of the invention further comprise the administration of one or more other agents selected from tenofovir DF (TDF), emtricitabine (FTC), zidovudine (AZT), didanosine (ddI), stavudine (d4T), abacavir (ABC), atazanavir (ATV), lopinavir (LPV), saquinavir (SQV), tipranavir (TPV), fosamprenavir (FosAPV) and efavirenz (EFV). The kits of the invention can also optionally further comprise one or more other agents selected from the above list.

The present invention also provides a kit comprising (i) an integrase inhibitor (e.g. Compound **1**), or a pharmaceutically acceptable salt thereof, (ii) ritonavir, or a pharmaceutically acceptable salt thereof, (iii) prescribing information, and (iii) one or more containers. The prescribing information may provide prescribing information conforming to the methods of the invention and/or as otherwise discussed herein. In an embodiment of the invention, the prescribing information includes administering the integrase inhibitor or a pharmaceutically acceptable salt thereof with ritonavir to improve the pharmacokinetics of the integrase inhibitor.

The effects of ritonavir on the bioavailability of a representative integrase inhibitor will now be illustrated by the following non-limiting Examples.

### Example 1. Effects of Ritonavir Boosting on the Pharmacokinetics of Compound 1

The effects of coadministration of 100 mg ritonavir (RTV) on the steady-state pharmacokinetics of Compound **1** were determined. The single-dose and multiple-dose pharmacokinetics of Compound **1** were also determined. The multiple-dose safety of Compound **1** administered alone and with RTV was also determined.

### Methods

The study was an open-label, fixed-sequence, crossover, pharmacokinetic study with 12 subjects. The subjects were healthy males and non-pregnant, nonlactating females between 18 and 45 years of age, inclusive.

The duration of the study was 20 days, with Period 1 of Days 1 to 10 and Period 2 of days 11 to 20. Follow-up contact was on day 27.

Compound **1** (100 mg) and RTV (100 mg) were administered twice daily, orally, immediately after a meal. Compound **1** (100 mg) was administered twice daily, orally, immediately after a meal.

### Criteria for Evaluation

**Pharmacokinetics:** The following parameters were calculated for Compound **1** (and metabolites, if possible) in plasma: Cₘₐₓ, Tₘₐₓ, Cₗₐₛₜ, Tₗₐₛₜ, Cₜₐᵤ, λ_{z}, AUC₀₋ₗₐₛₜ, AUC_{inf}, %AUCₑₓₚ, AUCₜₐᵤ, T_{½}, V_{z}/F, and CL/F.

**Safety:** Safety was evaluated by assessment of clinical laboratory tests at baseline and at various time points during the study; periodic physical examinations, including vital signs; and documentation of adverse events throughout the study.

### Statistical Methods

**Pharmacokinetics:** The pharmacokinetics of Compound **1** and RTV were summarized using descriptive statistics. In addition, a parametric (normal theory) analysis of variance (ANOVA) using a mixed-effects model appropriate for a crossover design was fit to the natural logarithmic transformation of Compound **1** pharmacokinetic parameters (AUC and Cₘₐₓ). Comparisons of multiple-dose to single-dose pharmacokinetics of Compound **1** and multiple-dose pharmacokinetics of Compound **1** with and without RTV were performed using 90% confidence intervals for the ratio of geometric means for each treatment pair.

**Safety:** No statistical inference was performed for safety data in this pharmacokinetic study.

### RESULTS

**Pharmacokinetic Results:** Mean (%CV) plasma pharmacokinetic parameters of Compound **1** and RTV after single oral dosing of Compound **1** (100 mg, Day 1) and multiple oral dosing of Compound **1** (100 mg twice daily, Day 10) in the absence of RTV or in the presence of RTV administered orally as a single dose (100 mg, Day 11) or multiple doses (100 mg twice daily, Day 20) were as follows.

| **Parameters** | **Compound 1 Alone (N=12)** | | **Compound 1 + RTV (N=12)** | |
|---|---|---|---|---|
| | **Day 1** | **Day 10** | **Day 11** | **Day 20** |
| **Compound 1** | | | | |
| AUC (ng•h/mL)^{a} | 908.1 (28.3) | 719.3 (26.2) | 6167.3 (29.1) | 14302.1 (23.7) |
| Cₘₐₓ (ng/mL) | 200.1 (30.4) | 164.1 (28.8) | 795.3 (38.4) | 1826.4 (26.4) |
| Cₜₐᵤ (ng/mL)^{b} | 19.2 (52.5) | 12.4 (63.7) | 543.3 (30.4) | 1035.6 (32.0) |
| T_{½} (h)^{c} | 3.1 (2.2, 4.8) | 3.5 (2.2, 4.1) | 18.2 (9.0,42.6) | 9.5 (5.9, 78.2) |

| **Ritonavir** | | | | |
|---|---|---|---|---|
| AUC (ng•h/ml)^{d} | Not applicable | Not applicable | 4979.4 (57.8) | 9402.5 (46.9) |
| Cₘₐₓ (ng/mL) | Not applicable | Not applicable | 616.3 (53.5) | 1686.5 (46.5) |
| Cₜₐᵤ (ng/ml)^{b} | Not applicable | Not applicable | 219.8 (61.8) | 544.8 (44.3) |
| T_{½} (h)^{c} | Not applicable | Not applicable | 5.1 (2.2, 8.3) | 4.8 (4.3, 6.9) |

| | | | | |
|---|---|---|---|---|
| a For Compound 1, AUC represents AUC_{inf} on Day 1 and AUCₜₐᵤ. on Days 10, 11, and 20. b Cₜₐᵤ represents the concentration at the end of the dosing interval for Days 1, 10, 11, and 20. c Median (min, max) d For ritonavir, AUC represents AUC_{inf} on Day 11 and AUCₜₐᵤ. on Day 20. | | | | |

During administration of Compound **1** alone, the mean steady-state systemic exposure (AUCₜₐᵤ) to Compound **1** (Day 10) was approximately 20% lower compared with the mean exposure (AUC_{inf}) after a single dose (Day 1), indicating autoinduction of Compound **1** metabolism. Coadministration with RTV resulted in a net inhibition of Compound **1** metabolism, as evidenced by both greater-than-predicted steady-state exposures and a relatively long median elimination half-life (9.5 vs. 3.5 hours, at steady state). The increase in Compound **1** exposure after coadministration of RTV is likely due to a combination of improved oral bioavailability due to decreased first-pass metabolism, with a component of reduced systemic clearance, as indicated by a change in observed T_{½}.

Overall, these data support the use of RTV (*e.g*., low-dose RTV) as a pharmacokinetic booster of Compound **1** to enable, *e.g*., achievement of higher trough concentrations and less frequent dosing intervals.

**Safety Results:** Treatment-emergent adverse events (AEs) were reported for 4 of 12 subjects (33%, five events) during administration of Compound **1** alone and for 7 of 12 subjects (58%, 44 events) during administration of Compound **1**+RTV. No single AE was reported in more than one subject during administration of Compound **1** alone. During administration of Compound **1**+RTV, the most frequently reported treatment-emergent AE was nausea, experienced by 4 subjects (33%). Most treatment-emergent AEs were mild (Grade 1) in severity and resolved without therapy.

Treatment-emergent AEs considered by the investigator to be related to study drugs were reported in 2 of 12 subjects (17%, two events) after administration of Compound **1** alone and in 5 of 12 subjects (42%, 21 events) after administration of Compound **1**+RTV. No treatment-related AE was reported in more than one subject after administration of Compound **1** alone. Treatment-related AEs reported in more than one subject after administration of Compound **1**+RTV were nausea (three subjects), vomiting (two subjects), headache (two subjects), and pruritus (two subjects).

No serious adverse events occurred, no subject discontinued because of an adverse event, and no pregnancies occurred during this study. No subject discontinued study drugs because of a clinical laboratory abnormality, and no clinical laboratory abnormality was reported as an AE.

### CONCLUSIONS

Coadministration with RTV resulted in a net inhibition of Compound **1** metabolism and significantly increased systemic exposures, in particular, trough concentrations. The data support the use of low-dose RTV as a pharmacokinetic booster of Compound **1**.

Oral administration of Compound 1 (100 mg twice daily) for up to 20 days was well tolerated in study subjects. After administration of Compound **1** alone, all adverse events were mild and transient. Concurrent oral administration of Compound **1** (100 mg twice daily) and RTV (100 mg twice daily) for up to 10 days was generally well-tolerated in study subjects. After administration of Compound **1**+RTV, most adverse events reported were mild and transient and generally consistent with those reported for RTV.

### Example 2. Safety, Pharmacokinetics, and Antiviral Activity of Compound 1 Following Oral Administration in Subjects Infected with HIV-1

The safety, tolerability, and antiviral activity of Compound **1** administered orally as 10 consecutive daily doses (twice-daily for Cohorts 1, 2, and 4; once-daily for Cohorts 3 and 5) in subjects chronically infected with HIV-1 not currently receiving antiretroviral therapy were investigated. The pharmacokinetics and pharmacodynamics of Compound **1** were also investigated.

### Methods

The studies are double-blind, randomized, placebo-controlled, sequential-cohort, dose-ranging, Phase 1/2 studies of Compound 1 therapy in antiretroviral-naïve- or - experienced HIV-infected adults who were not currently receiving antiretroviral therapy. At screening, subjects were to have a plasma HIV-1 RNA load of ≥ 10,000 to ≤ 300,000 copies/mL and a CD4+ cell count of ≥ 200 cells/mm³.

Five successive cohorts of 8 unique subjects (6 active and 2 placebo subjects) were treated for 10 consecutive days starting on Day 1 with study drug or placebo in the fed state. Safety, tolerability, pharmacokinetics, and efficacy were monitored through Day 21 after dosing. The active treatments in the 5 cohorts were as follows: Cohort 1, 400 mg of Compound **1** twice-daily; Cohort 2, 800 mg of Compound **1** twice-daily; Cohort 3, 800 mg of Compound **1** once-daily; Cohort 4, 200 mg of Compound **1** twice-daily; Cohort 5, 50 mg of Compound **1**+100 mg of RTV (RTV), each once daily. The placebo group in Cohort 5 also received 100 mg of RTV.

Eight subjects were enrolled per cohort, 2 placebo and 6 active (randomized: 48, dosed 40). Safety was evaluated for 8 subjects per cohort (2 placebo, 6 active), total 40 subjects. Pharmacokinetics were evaluated for 28 subjects on Day 1, 30 subjects on Day 10. Pharmacokinetics were evaluated for 30 subjects, and HIV-1 RNA was evaluated for 40 subjects. Efficacy was determined for 8 subjects per cohort (2 placebo, 6 active), total 40 subjects.

Subjects were males and females aged 18 to 60 years, inclusive, who were chronically infected with HIV-1 and not currently receiving antiretroviral therapy with a screening plasma HIV-1 RNA of ≥ 10,000 to ≤ 300,000 copies/mL and a screening CD4+ cell count of ≥ 200 cells/mm³. Subjects may have been antiretroviral treatment-experienced or -naive, but were not to have received antiviral medication within 90 days of baseline (Day 0).

The duration of the study was 21 days, with 10 days of treatment and 11 days of follow-up. 50 mg or 200 mg Compound 1 tablets were administered orally under fed conditions. Matching placebo was administered orally under fed conditions. For Cohort 5 only, 100 mg RTV capsules were coadministered orally under fed conditions.

### Criteria for Evaluation

Efficacy: The primary efficacy endpoint was the maximum HIV-1 RNA reduction (log₁₀ copies/mL) from baseline through Day 11. The maximum reduction for an individual subject was the maximum drop among changes from baseline between Days 2 and 11.

Pharmacokinetics: The following plasma pharmacokinetic parameters for Compound **1** were calculated: Cₘₐₓ, Tₘₐₓ, Cₗₐₛₜ, Tₗₐₛₜ, Cₜₐᵤ, λ_{z}, T_{½}, AUC₀₋ₗₐₛₜ, AUC_{inf}, % AUCₑₓₚ, AUCₜₐᵤ, V_{z}/F, and CL/F. Concentrations of Compound **1** in peripheral mononuclear cells (PBMC) were determined and the pharmacokinetics of Compound **1** in PBMCs was explored.

Safety: Adverse events, vital signs, electrocardiogram, ophthalmologic examination, and clinical laboratory tests were evaluated for safety.

### Statistical Methods

**Efficacy:** Pairwise comparisons were conducted using Wilcoxon rank sum exact test to compare the placebo group (2 subjects each from Cohorts 1 through 4 combined) and each of the 5 Compound **1** dose levels (6 subjects at each dose level) and between each pair among the 5 Compound **1** dose levels. The 2 subjects who received placebo + 100 mg RTV were treated as a separate group. HIV-1 RNA values below the detection limit (50 copies/mL) were entered as 49 copies/mL.

**Pharmacokinetics:** The pharmacokinetic parameters of Compound **1** in plasma and peripheral blood mononuclear cells were summarized using descriptive statistics. In addition, for each Compound **1** dose level, an analysis of variance was performed on pharmacokinetic parameters (AUC and Cₘₐₓ) to examine dose proportionality and steady-state pharmacokinetics. Compound **1** trough levels measured on Days 2, 4, 7, 10, and 11 were included, as appropriate, in these analyses.

**Safety:** The proportion of subjects with adverse events was summarized by treatment, system organ class and preferred term for treatment-emergent adverse events, treatment-related adverse events, serious adverse events, and adverse events leading to study drug discontinuation. Adverse events were coded using the Medical Dictionary for Regulatory Activities (MedDRA^{®}) version 8.1. Additional summaries of adverse events were displayed by highest grade, investigator's assessment of relationship to study drug, and effect on study drug discontinuation. Laboratory results were expressed on the original measurement scale and in terms of the toxicity grade. Changes from baseline in quantitative laboratory tests were summarized by visit.

### RESULTS

**Efficacy, Pharmacokinetic, and Pharmacodynamic Results** The steady-state pharmacokinetic parameters (mean [% CV]) and antiviral activity of Compound 1 when dosed alone (200 mg twice-daily, 400 mg twice-daily, 800 mg twice-daily, or 800 mg once-daily) or 50 mg once-daily coadministered with 100 mg of RTV are presented in the following table:

| **Parameter** | **Placebo (N = 8)** | **Placebo/r (N = 2)** | **Compound 1 200 mg BID (N = 6)** | **Compound 1 400 mg BID (N = 6)** | **Compound 1 800 mg QD (N = 6)** | **Compound 1 800 mg BID (N = 6)** | **Compound 1 50 mg QD + 100 mg RTV (N = 6)** |
|---|---|---|---|---|---|---|---|
| Maximum Reduction in HIV-1 RNA (log₁₀ copies/mL)^{a} | | | | | | | |
| Mean (SD) | -0.25 (0.15) | -0.05 (0.14) | -1.48 (0.55)^{b} | -1.94 (0.52)^{b} | -0.98 (0.37)^{b, c} | -1.91 (0.60)^{b} | -1.99 (0.38)^{b ,d} |
| Median (min, max) | -0.26 (-0.48, 0.01) | -0.05 (-0.15, 0.05) | -1.48 (-2.10, -0.87) | -2.03 (-2.44, -1.04) | -0.96 (-1.41, -0.56) | -1.78 (-2.67, -1.27) | -2.03 (-2.38, -1.54)) |
| Subjects Achieving HIV-1 RNA < 50 copies/mL After Baseline | | | | | | | |
| n (%)^{e} | 0 (0%) | 0 (0%) | 0 (0%) | 0 (0%) | 0 (0%) | 2 (33%) | 0 (0%) |
| Subjects Achieving HIV-1 RNA < 400 copies/mL After Baseline | | | | | | | |
| n (%)^{e} | 0 (0%) | 0 (0%) | 0 (0%) | 0 (0%) | 0 (0%) | 3 (50%) | 2 (33%) |
| Compound 1 Steady-State Pharmacokinetics^{f} | | | | | | | |
| AUCₜₐᵤ (ng•h/mL) (mean, % CV) | - | - | 1954.65 (46.35) | 2335.30 (54.52) | 5512.87 (53.59) | 3566.35 (36.83) | 8843.50 (25.46) |
| Cₘₐₓ (ng/mL) (mean, % CV) | - | - | 479.03 (42.58) | 606.87 (77.58) | 939.92 (54.31) | 835.53 (48.20) | 744.65 (20.40) |
| Cₜₐᵤ (ng/mL) (mean, % CV) | - | - | 30.73 (39.98) | 48.68 (64.84) | 13.62 (68.64) | 47.98 (32.65) | 135.00 (36.55) |
| T_{½} (h) (median) (min, max) | - | - | 2.82 (2.51,4.75) | 3.08 (2.48, 5.02) | 3.80 (3.02, 4.60) | 2.53 (2.14,3.03) | 8.86 (6.10, 10.91) |

| | | | | | | | |
|---|---|---|---|---|---|---|---|
| a The maximum reduction in HIV-1 RNA was defined as the maximum decrease from baseline in log₁₀ copies/mL between Days 2 and 11. HIV-1 RNA values below the limit of quantitation (50 copies/mL) were entered as 49 copies/mL. The 5 Compound 1 dose levels and placebo + RTV group were compared versus the placebo group. b p = 0.0007 for each of the Compound 1 dose levels versus placebo based on pairwise p-values for continuous data from a 2-sided Wilcoxon rank sum exact test. c p = 0.0152 for the 800 mg of Compound 1 once-daily group versus 800 mg of Compound 1 twice-daily dose level and versus 400 mg of Compound 1 twice-daily based on pairwise p-values for continuous data calculated by a 2-sided Wilcoxon rank sum exact test. d The 50 mg of Compound 1 + RTV dose level once-daily versus placebo + RTV, p = 0.0714; versus 200 mg twice-daily, p = 0.1797; versus 400 mg twice-daily, p = 0.9372; versus 800 mg once-daily, p = 0.0022; versus 800 mg twice-daily, p = 0.8182. e The percentage is based on all randomized and treated subjects. f The 12- and 24-hour pharmacokinetic samples corresponding to the end of the dosing interval (tau) collected on Days 10 (twice-daily dosing) or 11 (once-daily dosing) were assigned nominal times of 12 or 24 hours, respectively. | | | | | | | |

Compound **1** monotherapy significantly reduced HIV-1 RNA levels at all dose levels compared with placebo (p < 0.0007). During 10 consecutive days of dosing, Compound **1** doses of 400 mg twice-daily, 800 mg twice-daily, or 50 mg coadministered with 100 mg of RTV once-daily resulted in maximum reductions in HIV-1 RNA from baseline of -1.94 ± 0.52, -1.91 ± 0.60 and -1.99 ± 0.38 log₁₀ copies/mL (mean ± SD), respectively. After 10 days of monotherapy with Compound **1**, no subject developed HIV-1 integrase mutations that corresponded to Compound **1** resistance mutations observed in selection experiments in vitro or to mutations that developed under selection with other experimental integrase inhibitors.

Compound **1** did not exhibit dose-proportional increases in the pharmacokinetics with ascending doses (200, 400, and 800 mg twice-daily) and demonstrated dose-dependent autoinduction of metabolism. Coadministration of a 50 mg dose of Compound **1** + 100 mg of RTV once-daily resulted in net inhibition of CYP3A-mediated metabolism and high systemic exposures, in particular trough concentrations. Pharmacokinetic findings in PBMCs were consistent with plasma pharmacokinetic data. A Compound **1** exposure-response relationship was identified with Compound **1** Cₜₐᵤ values that fit well to a simple Cₘₐₓ model with an EC₅₀ of 14.4 ng/mL and an Eₘₐₓ of 2.32 log₁₀ copies/mL reduction from baseline. The estimated inhibitory quotient of Compound **1**, calculated as the observed mean Cₜₐᵤ divided by the protein-binding adjusted, in vitro IC₅₀ of 7.17 ng/mL, was 5.9, 6.7, and 18.8 at the 400 mg twice-daily, 800 mg twice-daily, and 50 mg + RTV once-daily dose levels, respectively. Compound **1** trough concentrations at these doses also exceeded the protein binding-adjusted in vitro IC₉₅ of 44.9 ng/mL (100 nM) for the entire dosing interval.

**Safety Results** Treatment-emergent adverse events were experienced by most subjects, but headache and diarrhea were the only adverse events reported by more than 2 subjects within a group. The incidence of treatment-emergent adverse events in subjects who received Compound **1** was similar to, or lower than, the incidence in the placebo groups, and the classes of adverse events were similar. No adverse event that was considered to be related to study drug by the investigator was experienced by more than 2 subjects within a group, and only 3 treatment-related adverse events by preferred term occurred in 2 subjects within a group: diarrhea (placebo), nausea, (placebo and 200 mg of Compound **1** twice-daily), and fatigue (200 mg of Compound **1** twice-daily).

All 40 subjects who received study drug completed the study. There were no dose interruptions, discontinuations, or serious adverse events. Five subjects had treatment-emergent Grade 3 or 4 laboratory abnormalities, 2 subjects in the placebo group and 1 subject in each of the placebo + RTV once-daily, the 400 mg of Compound **1** twice-daily, and 50 mg of Compound **1** + RTV once-daily groups. One of the Grade 3 laboratory abnormalities in the placebo group, high triglycerides that resolved without therapy, was considered by the investigator to be an adverse event that was unrelated to study drug. The 2 subjects treated with Compound **1** who had Grade 3 laboratory abnormalities (elevated nonfasting triglycerides or elevated serum amylase), also had abnormal values at baseline. No clinically relevant changes in hematology and urinalysis findings, vital signs, body weight, or electrocardiogram and ophthalmic examinations occurred during the study.

### CONCLUSIONS

**Efficacy:** Administration of Compound **1** at doses of 200, 400, or 800 mg twice-daily; 800 mg once-daily, or 50 mg + 100 mg RTV once-daily to antiviral-naive or -experienced HIV-infected subjects for 10 consecutive days as monotherapy significantly reduced HIV-1 RNA compared with placebo at all dose levels. Compound **1** doses of 400 mg twice-daily, 800 mg twice-daily, and 50 mg + 100 mg RTV once-daily resulted in mean declines from baseline of -1.94, -1.91 and -1.99 log₁₀ copies/mL, respectively. No Compound **1** resistance mutations were detected in the integrase gene of any subject through Day 21.

**Pharmacokinetics/Pharmacodynamics:** Compound **1** exhibited pharmacokinetics supporting twice-daily dosing alone or once-daily dosing with a low dose (100 mg) of RTV. A Compound **1** exposure-response relationship was identified with Compound **1** Cₜₐᵤ values that fit well to a simple Eₘₐₓ model. The estimated inhibitory quotient of Compound **1**, calculated using a protein-binding adjusted in vitro IC₅₀ of 7.17 ng/mL, was 5.9, 6.7, and 18.8 at the 400 mg twice-daily, 800 mg twice-daily, and 50 mg + RTV dose once-daily levels, respectively. Compound **1** trough concentrations at these dose levels exceeded the protein binding-adjusted in vitro IC₉₅ of 44.9 ng/mL for the entire dosing interval.

**Safety:** Compound **1** was generally well tolerated at all dose levels, with no dose interruptions, discontinuations, or serious adverse events. Most adverse events were mild and resolved with therapy. The most common treatment-emergent adverse event was headache, and the most common treatment-related adverse event was nausea. The classes, frequency, and severity of adverse events and laboratory abnormalities were similar between the active and placebo groups.

### Example 3. Effects of Ritonavir Doses on the Pharmacokinetics of Compound 1

The effects of a range ofritonavir (RTV) doses (20, 50, 100, and 200 mg once daily) on the pharmacokinetics of Compound **1** were evaluated. The effects of a range of RTV doses (20, 50, 100, and 200 mg once daily) on hepatic cytochrome P450 3A (CYP3A) activity were also evaluated using a CYP3A substrate. The safety and tolerability of a range of RTV doses in combination with Compound **1** were also evaluated.

### METHODS

A randomized , open label, single-center, multiple-dose, two group, Phase 1 study was conducted (24 subjects (16 evaluable) subjects in two groups; 12 (8 evaluable) in each group) with an approximately even distribution of healthy male and non-pregnant, non-lactating female subjects aged between 18-45, inclusive

Eligible subjects were males and non-pregnant, non-lactating females, with a body mass index (BMI) 19 ≤ BMI ≤ 30, no significant medical history and in good general health as determined by the investigator at screening evaluation performed no more than 28 days prior to the scheduled first dose. The estimated creatinine clearance by Cockroft-Gault (using actual body weight) was be ≥ 80 mL/min.

The following Compound **1**/r study treatments were used:
Treatment A: Compound **1**, 1 × 125 mg tablet plus RTV 20 mg (80 mg/mL solution diluted to give 20 mg dose) QD (both drugs in the AM).
Treatment B: Compound **1**, 1 × 125 mg tablet plus RTV 50 mg (80 mg/mL solution diluted to give 50 mg dose) QD (both drugs in the AM).
Treatment C: Compound **1**, 1 × 125 mg tablet plus RTV 100 mg (80 mg/mL solution used to give 100 mg dose) QD (both drugs in the AM).
Treatment D: Compound **1**, 1 × 125 mg tablet plus RTV 200 mg (80 mg/mL solution used to give 200 mg dose) QD (both drugs in the AM).

For each study treatment, RTV was administered first, immediately followed by Compound **1**.

A slow IV push of midazolam (MDZ; 1 mg over 1 minute), as a probe for CYP3A activity, was administered to each subject at 14:00 on Day 1 and 6 hours post- Compound **1**/r dosing on Days 11 and 21.

Following screening procedures and baseline assessments, eligible subjects were randomized to one of two treatment groups as described below:

| **Day** | **Treatment and/or MDZ Probe** | **PK Assessment conducted** |
|---|---|---|
| **Group 1** | | |
| 1 | MDZ | MDZ |
| 2-10 | A: in the AM | None |
| 11 | A: in the AM MDZ: 6 hours postdose | Compound 1, RTV and MDZ |
| 12-20 | C: in the AM | None |
| 21 | C: in the AM MDZ: 6 hours postdose | Compound 1, RTV and MDZ |
| | | |

| **Group 2** | | |
|---|---|---|
| 1 | MDZ | MDZ |
| 2-10 | B: in the AM | None |
| 11 | B: in the AM MDZ: 6 hours postdose | Compound 1, RTV and MDZ |
| 12-20 | D: in the AM | None |
| 21 | D: in the AM MDZ: 6 hours postdose | Compound 1, RTV and MDZ |

All Compound **1**/r doses were administered immediately after completion of an AM meal with 240 mL of water. Serial blood samples for analysis of Compound **1** and RTV plasma concentrations were collected on Days 11 and 21 at the following time points post- Compound **1**/r dosing: 0 (predose), 0.5, 1, 1.5, 2, 3, 3.5, 4, 4.5, 5, 5.5, 6, 8, 10, 12, 14, 16, 18, and 24 hours postdose. Serial blood samples for analysis of MDZ plasma concentrations were collected on Days 1, 11, and 21 at the following time points post- MDZ dosing: 0 (predose), 5 minutes, 10 minutes, 15 minutes, 30 minutes and 1, 2, 4, 6, 8, 10, 12, and 18 hours after MDZ administration. Pharmacokinetic parameters of Compound **1**, RTV and MDZ were estimated.

An ECG assessment was performed at Screening. Clinical laboratory tests, physical examinations and vital signs (temperature, blood pressure, heart rate, and respiration rate) were performed at Screening, Baseline and on Days 10 and 20. Vital signs (temperature, blood pressure, heart rate, and respiration rate) were also performed on Days 1, 11 and 21 at 0 hour (pre-midazolam dose) and at 0.5, 1.0, 1.5, and 2.0 hours post-midazolam dose.

### Test Product, Dose, and Mode of Administration:

1. Compound **1**, 1 × 125 mg tablet plus RTV 20 mg QD (in the AM)
2. Compound **1**, 1 × 125 mg tablet plus RTV 50 mg QD (in the AM)
3. Compound **1**, 1 × 125 mg tablet plus RTV 200 mg QD (in the AM)

All Compound **1**/r doses were administered immediately following completion of a standardized meal.

### Reference Therapy, Dose, and Mode of Administration

Compound **1**, **1** × 125 mg tablet plus RTV 100 mg QD (in the AM) All Compound **1**/r doses were administered immediately following completion of a standardized meal.

### Criteria for Evaluation:

Safety was evaluated by assessment of clinical laboratory tests, ECG, periodic physical examinations including vital signs at various time points during the study, and by the documentation of adverse events.

The following plasma pharmacokinetic parameters were calculated: For Compound **1** and RTV: Cₘₐₓ, Tₘₐₓ, Cₗₐₛₜ, Tₗₐₛₜ, Cₜₐᵤ, λ_{z}, , AUCₜₐᵤ, T_{½}, CL/F and Vz/F For MDZ: AUC₀₋ₗₐₛₜ, AUC_{inf}, %AUCₑₓₚ, Cₗₐₛₜ, Tₗₐₛₜ, λ_{z}, T_{½}, CL and Vz.

**Statistical Methods:** Safety and pharmacokinetic parameters for Compound **1,** RTV and MDZ were summarized by subject and RTV dose level using descriptive statistics. Power models and/or ANOVA models (as appropriate) were fitted using Compound **1** pharmacokinetic parameters (AUC, Cₘₐₓ and Cₜₐᵤ) from all treatments. The population mean slope was estimated along with a corresponding 90 % CI. This study was performed in compliance with the guidelines of Good Clinical Practices (GCPs).

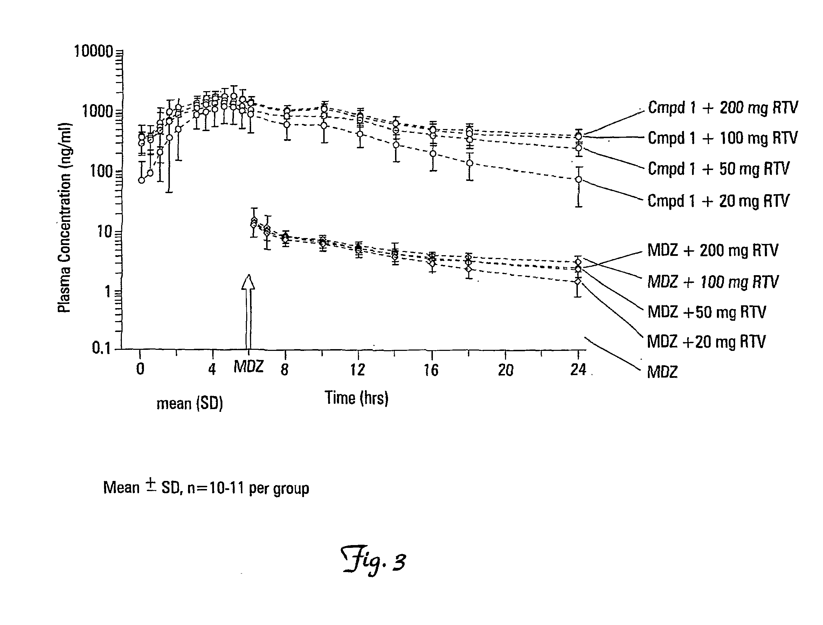

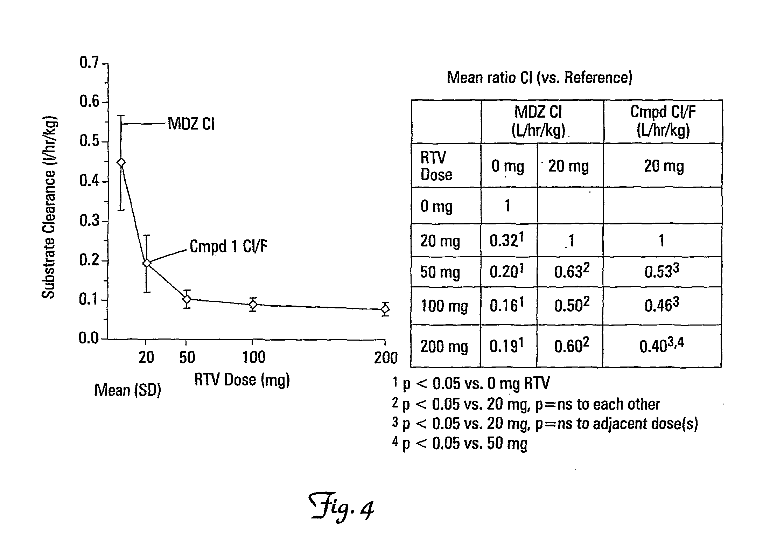

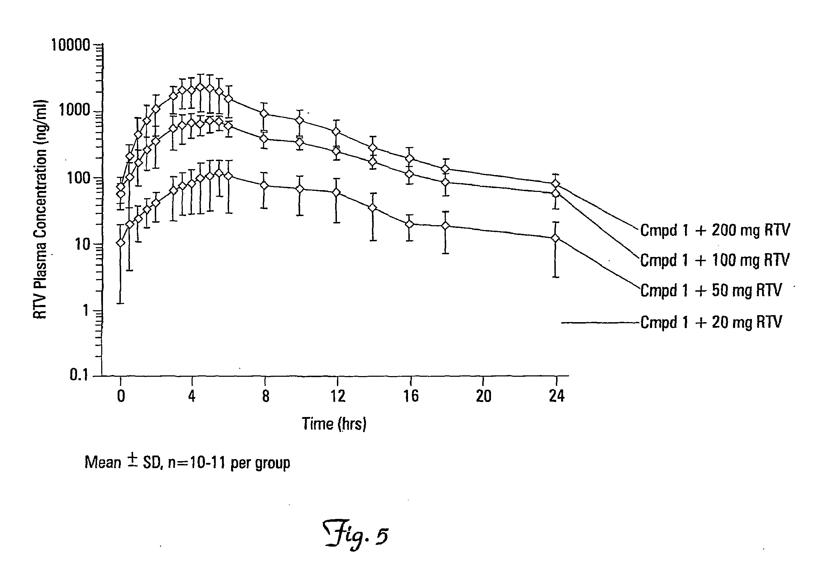

**Results:** Results from the study are shown in the following three Tables 3A-3C and in Figures 3-5.

**Table 3A: Mean (CV%) MDZ PK Parameters**

| PK Parameter | MDZ Alone | + 20 mg RTV | + 50 mg RTV | +100 mg RTV | + 200 mg RTV |
|---|---|---|---|---|---|
| AUC_{inf} (ng.hr/ml) | 31.0 (30.9) | 101 (28.7) | 145 (36.1) | 219 (49.0) | 146 (22.6) |
| Cₗₐₛₜ (ng/ml) | 0.20 (40.6) | 1.53 (42.4) | 2.51 (26.7) | 3.34 (23.1) | 2.58 (24.8) |
| Half-life (hr) | 3.97 (34.4) | 7.83 (38.9) | 14.4 (70.4) | 22.4 (70.7) | 15.2 (31.6) |
| CL (1/hr/kg) | 0.447 (27.9) | 0.143 (26.5)¹ | 0.090 (25.2)^{1,2} | 0.072 (33.6)^{1,2} | 0.087 (22.0)^{1,2} |

| | | | | | |
|---|---|---|---|---|---|
| ¹ All +RTV doses different than MDZ alone (p<0.05) ^{1,2} p<0.05 vs. 20 mg, p=ns to each other | | | | | |

**Table 3B: Mean (CV%) Compound 1 Parameters**

| Parameter | Compound **1** + 20 mg RTV | Compound **1** + 50 mg RTV¹ | Compound **1** + 100 mg RTV¹ | Compound **1** + 200 mg RTV¹ |
|---|---|---|---|---|
| AUCₜₐᵤ (ng.hr/ml) | 10200 (36.3) | 15800 (24.4) | 20300 (24.8)² | 20600 (24.3)² |
| Cₜₐᵤ (ng/ml) | 73.8 (60.2) | 251 (27.7) | 380 (39.9) | 410 (26.0) |
| Cₘₐₓ (ng/ml) | 1370 (43.0) | 1560 (36.4) | 1830 (20.1) | 2030 (37.4) |
| Half-life (hr) | 4.34 (34.1) | 9.52 (27.5) | 11.5 (28.8) | 14.3 (27.6)² |
| IQ | 10.3 | 35.0 | 53.0 | 57.2 |
| Fold IC₉₅ | 1.65 | 5.60 | 8.48 | 9.15 |

| | | | | |
|---|---|---|---|---|
| IQ and fold IC₉₅ based on protein binding-adjusted IC₅₀ and IC₉₅ in PBMCs ¹ RTV 50, 100, and 200 mg different from 20 mg for all PK parameters ² p < 0.05 vs. 50 mg | | | | |

**Table 3C: Mean (CV%) RTV PK Parameters**

| PK Parameter | Compound **1** + 20 mg RTV | Compound **1** + 50 mg RTV | Compound **1** + 100 mg RTV | Compound **1** + 200 mg RTV |
|---|---|---|---|---|
| AUCₜₐᵤ (ng.hr/ml) | 135 (54.9) | 1120 (61.4) | 6550 (26.9) | 16000 (43.9) |
| Cₜₐᵤ (ng/ml) | 0.718 (98.8) | 11.6 (66.2) | 53.8 (41.6) | 78.5 (36.7) |
| Cₘₐₓ (ng/ml) | 19.5 (56.7) | 130 (61.3) | 807 (29.5) | 2460 (50.5) |
| Half-life (hr) | 4.74 (34.3) | 6.54 (32.6) | 6.34 (18.4) | 5.71 (15.9) |

| | | | | |
|---|---|---|---|---|
| · RTV exposures not dose-proportional, very low exposures at 20 mg with BLQ concentrations (< 0.5 ng/ml) by 16 hr in some subjects · T_{1/2} and MDZ clearance data suggest dose-dependent RTV first-pass · Higher CV% at doses <100 mg | | | | |

### Example 4 Co-administration of Emtricitabine/Tenofovir Disoproxil Fumarate and Ritonavir-boosted Compound 1

Once-daily administration of Compound **1** with low dose ritonavir results in net inhibition of metabolism in addition to a marked enhancement of exposure (10 to 16-fold). Additionally, once-daily ritonavir-boosted Compound **1** achieves high trough concentrations resulting in an inhibitory quotient (using protein-binding adjusted IC₅₀) ≥3-fold greater compared to unboosted dosing regimens of Compound **1,** thereby offering a degree of forgiveness for late or missed doses coupled with a convenient dosing regimen for patients. As a novel agent from a new therapeutic class with potent short-term activity, favorable safety profile, and a simple once-daily dosing schedule to support regimen-adherence, ritonavir-boosted Compound **1** offers tremendous potential for HIV treatment, including patients that are multiple class-experienced.

The fixed-dose combination of emtricitabine/tenofovir disoproxil fumarate (FTC/TDF) is a simple, highly efficacious NRTI combination, with superior safety profile compared to thymidine analogs, and recommended by United States Department of Health and Human Services as a preferred nucleos(t)ide backbone for treatment-naïve and treatment-experienced adult and adolescent HIV patients. The present study was performed in part to assess the potential for a clinically relevant drug-drug interaction upon their co-administration.

The use of healthy subjects in this study removed potential confounding factors associated with a background regimen and avoided short-term changes in treatment regimen in HIV patients for the purpose of evaluating a pharmacokinetic (PK) interaction.

Thus, the potential for clinically relevant drug-drug interactions between emtricitabine/tenofovir disoproxil fumarate (FTC/TDF) and the HIV integrase inhibitor, ritonavir-boosted Compound **1** (Compound **1**/r) were examined.

Healthy adults were administered FTC/TDF (200mg/300mg; once-daily) for 7 days, followed by randomization to the order of receiving Compound **1**/r (50mg/100mg; once-daily) and Compound **1**/r plus FTC/TDF in a crossover fashion, under fed conditions for 10 days. Pharmacokinetic (PK) blood draws were performed on days 7, 17, and 27. Lack of PK alteration for FTC, tenofovir (TFV), and Compound **1** was defined as 90% confidence intervals (CI) for the geometric mean ratio (GMR; co-administration:alone) between 70 to 143% for the primary PK parameters: maximum observed plasma concentration (Cₘₐₓ), area under the plasma concentration-time curve over dosing interval (AUCₜₐᵤ), and trough concentration (Cₜₐᵤ).

Twenty-four of the 26 enrolled subjects completed the study with no serious adverse events (serious AEs) or discontinuations due to AEs. FTC, TFV, and Compound **1** PK were unaffected during co-administration, with all three primary parameters meeting the protocol definition of equivalence and also the stricter bioequivalence criteria (90% CI: 80 to 125%). FTC and TFV PK parameters were comparable to historical values.

There was no clinically relevant drug-drug interaction between FTC/TDF and Compound 1/r upon their co-administration.

### Example 5 Lack of Clinically Relevant Drug-Drug Interaction Between the Ritonavir-Boosted HIV Integrase Inhibitor Compound 1/r and Zidovudine (ZDV)

Zidovudine is a NRTI used in HIV patients. The potential for drug interaction between zidovudine and Compound **1**/r was determined in a randomized study. Whether the pharmacokinetics of zidovudine or Compound **1** are affected after co-administration of zidovudine plus Compound **1**/r compared to administration alone was evaluated, Further, the safety of co-administration of zidovudine plus Compound **1**/r was evaluated.

As described below, there was no clinically relevant pharmacokinetic drug-drug interaction between zidovudine and Compound **1**/r. Co-administration of zidovudine and Compound **1**/r was safe and well tolerated. Compound **1** exhibited a half-life supporting once daily dosing that was sustained through 48 hours post-dosing.

Study drugs (Compound **1**/r 200/100 mg QD, ZDV 300 mg BID) were administered with a meal (- 400 kcal, 13 g fat). The pharmacokinetic (PK) sampling was done over 12 (ZDV) or 24 hours (Compound **1**/r). A 48 hour PK profile of Compound **1** was determined after Day 27 dosing. ZDV, G-ZDV and Compound **1** plasma levels were determined using validated LC/MS/MS assays. PK parameters were estimated using WinNonlin™ 5.0.1 (Pharsight Corporation, Mountain View, CA, USA). Equivalence bounds for 90% confidence interval (CI) were set at 70% to 143% (co-administration: alone) for geometric mean ratio (GMR) of AUCₜₐᵤ, Cₘₐₓ, and Cₜₐᵤ (Compound **1** only). **Demographics** 28 subjects were enrolled, and 24 subjects completed the study (12 males, 12 females; mean age: 31 yrs (range: 20 yrs to 42 yrs); mean weight: 70.2 kg (range: 50.9 kg to 101 kg); ethnicity: 20 Hispanic, 4 Caucasian).

**Safety** There were no grade 3/4 adverse events (AEs) or serious adverse events. There were four discontinuations: pregnancy (1), withdrawn consent (1), vomiting (1; ZDV + Compound **1**/r arm), abdominal distension (1; ZDV arm). Compound **1**/r and ZDV administered alone and in combination were generally well tolerated. The most frequent AEs across treatment arms were headache and nausea. All of the AEs were mild to moderate and resolved on treatment.

**Pharmacokinetic Results** ZDV, G-ZDV, and Compound **1** AUC_{tau,} C_{max,} Cₜₐᵤ (Compound **1** only) 90% CI were within predefined PK equivalence bounds, with ZDV and G-ZDV PK being similar to historical values.

### Food Effects

The present invention also provides methods for improving the pharmacokinetic properties of Compound **1** upon administration to a patient. The present invention also provides methods for improving the therapeutic effectiveness of Compound **1** for the treatment or prophylaxis of diseases, disorders, and conditions.

An example of a disease, disorder, or condition includes, but is not limited to, a retrovirus infection, or a disease, disorder, or condition associated with a retrovirus infection. Compound **1** may also be administered to a patient to inhibit the activity of a retrovirus integrase. In an embodiment of the invention, the retrovirus is HIV.

Pharmacokinetic studies have not previously been conducted to evaluate the effect of food on the pharmacokinetics of Compound **1.** In general, food has a variable effect on the bioavailability and absorption of an active agent. Drug-food interactions may result in reduced, delayed, or increased systemic drug availability. For example see Welling, Clin. Pharmacokinet., 9: 404-434 (1984).

It has been discovered that Compound **1** may be administered to patients in a method that increases the therapeutic effectiveness of Compound **1** in such patients. When administered with food, Compound **1** exhibits increased bioavailability and absorption in patients.

Accordingly, the invention provides a method of increasing the bioavailability and absorption of Compound **1** in a patient comprising administering to the patient a therapeutically effective amount of Compound **1** with food.

Compound 1 is described in United States Patent Application Serial Number 10/492,833, filed November 20, 2003 (U.S. Patent Application Publication No. 2005/0239819), and in United States Patent Application Serial Number 11/133,463, filed May 20, 2005 (U.S. Patent Application Publication No. 2005/0288326), which are herein incorporated by reference in their entirety. Compound **1** exists in at least three different crystal forms. Crystal forms I, II, and III are described in International Patent Application Publication Number WO 05/113508, which is also herein incorporated by reference in its entirety. These three forms are distinguishable by differential scanning calorimeter (DSC) and X-ray powder diffractometer (XRD). Any one of the crystal forms may be used in the present invention. In an embodiment of the invention, crystal form II or III, or a mixed crystal thereof, is administered to a patient.

While the administration of Compound **1** is a particular embodiment of the invention, the invention also contemplates the administration of other compounds that will yield Compound **1,** *e.g*., prodrugs of Compound **1.** Such prodrugs, for example, may include compounds that have protecting groups, but that still result in the formation of Compound **1** in the body of a patient (*i.e., in vivo*)*.* Carboxylic acid-protecting groups include, for example, alkyl esters and benzyl esters, which may be removed by an acid or base and hydrogenolysis, respectively. Moreover, a compound having any organic residue that may be dissociated *in vivo* to yield Compound **1** may be administered according to the method of the invention. Thus, the invention also contemplates the administration of Compound **1**' (wherien R' signifies an organic residue) so as to yield Compound **1.**

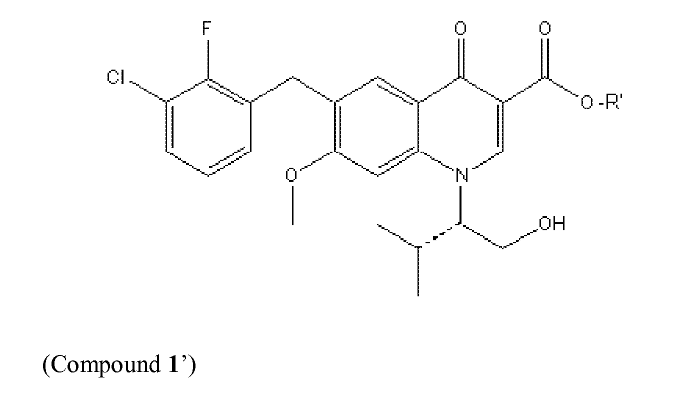

The present invention further contemplates the administration of a pharmaceutically acceptable salt of Compound **1**. For example, a pharmaceutically acceptable salt of Compound **1** may be obtained by reacting Compound **1** with: an inorganic acid such as hydrochloric acid, sulfuric acid, phosphoric acid, hydrobromic acid, and the like; an organic acid such as oxalic acid, malonic acid, citric acid, fumaric acid, lactic acid, malic acid, succinic acid, tartaric acid, acetic acid, trifluoroacetic acid, gluconic acid, ascorbic acid, methylsulfonic acid, benzylsulfonic acid, and the like; an inorganic base such as sodium hydroxide, potassium hydroxide, calcium hydroxide, magnesium hydroxide, ammonium hydroxide, and the like; an organic base such as methylamine, diethylamine, triethylamine, triethanolamine, ethylenediamine, tris(hydroxymethyl)methylamine, guanidine, choline, cinchonine, and the like; or an amino acid such as lysine, arginine, alanine, and the like. The present invention also encompasses the administration of water-containing products and solvates, such as hydrates, of Compound **1,** and the like.

Therefore, as used herein, "administering ... Compound **1**" refers to the administration of any form of Compound **1** that provides Compound **1** *in vivo.*

As used herein, the term "bioavailability" refers to the rate and extent to which the active ingredient is released from a drug product and becomes available at the site of action. *See* U.S. Code of Federal Regulations, Title 21, Part 320.1 (2001 ed.). For oral dosage forms, bioavailability relates to the processes by which the active ingredient is released from the oral dosage form, *e.g*., a tablet, and moved to the site of action, *e.g*., absorbed into the systemic circulation. Therefore, the term "absorption" refers to the presence of Compound **1** at its site of action, for example, in the bloodstream or in immunocytes, such as a T cell or macrophage.

As used herein, the term "with food" or "fed" refers to the condition of having taken food during the period between from about 1 hour prior to the administration of Compound **1** to about 2 hours after the administration of Compound **1.** In another embodiment, "with food" or "fed" refers to the condition of the administration of Compound **1** during the period between from about 1 hour prior to the consumption of food to about 2 hours after the consumption of food. "Food" as used herein, refers to both liquid and solid food. The food also can be low-fat diet, high-fat diet, light diet or heavy diet. In an embodiment of the invention, the food is a solid food with sufficient bulk and fat content that it is not rapidly dissolved and absorbed in the stomach. Food may be a meal, such as breakfast, lunch, or dinner. Additionally, food may be taken by mechanical administration, for example, intravenously, by involuntary consumption by the patient. Alternatively, the patient may voluntarily consume the food.

Compound **1** may be administered any time of the day with food. The food may typically be taken at any time during the period between from about 1 hour prior to the administration of Compound **1** to about 2 hours after the administration of Compound **1.** For example, the food may be taken within the time period of about 1 hour, about 45 minutes, about 30 minutes, about 15 minutes, about 10 minutes, or about 5 minutes prior to the administration of Compound **1.** Similarly, the food may be taken within the time period of about 5 minutes, about 10 minutes, about 15 minutes, about 30 minutes, about 45 minutes, about 1 hour, about 1.25 hours, about 1.5 hours, about 1.75 hours, or about 2 hours after the administration of Compound **1.** In another embodiment, "with food" or "fed" refers to the condition of the administration of Compound **1** during the period between from about 1 hour prior to the consumption of food to about 2 hours after the consumption of food. For example, Compound **1** may be administered within the time period of about 1 hour, about 45 minutes, about 30 minutes, about 15 minutes, about 10 minutes, or about 5 minutes prior to the consumption of food. Similarly, Compound **1** may be administered within the time period of about 5 minutes, about 10 minutes, about 15 minutes, about 30 minutes, about 45 minutes, about 1 hour, about 1.25 hours, about 1.5 hours, about 1.75 hours, or about 2 hours after the consumption of food. In another embodiment of the invention, Compound **1** may be administered immediately after food (e.g. within about 1 minute after food) up to about 1 hour after food. Ideally, Compound **1** is administered at substantially the same time as with the food.

As used herein, the terms "without food" or "fasted" refer to the condition of not having taken food within the time period of about 1 hour prior to the administration of Compound **1** to about 2 hours after the administration of Compound **1.**

The method of the invention comprises administering a therapeutically effective amount of Compound **1.** In the case of administering a single Compound **1** with food, a suitable dose of a therapeutically effective amount of Compound **1** for administration to a patient will be between approximately 10 mg to about 2000 mg per day. In an embodiment of the invention, a suitable dose is about 400 mg to about 1600 mg per day. In another embodiment of the invention, the suitable dose is about 600 mg to about 1200 mg per day. In a further embodiment of the invention, a suitable dose is about 800 mg per day.

If desired, the effective daily dose of Compound **1** may be administered as two, three, four, five, six, or more sub-doses administered separately at appropriate intervals throughout the day, optionally, in unit dosage forms. In accordance with the inventive method, Compound **1** may be administered with food at multiple times per day or, alternatively, once per day. In an embodiment of the invention, Compound **1** is administered with food once or twice per day.

As used herein, the term "unit dosage form" refers to the form of Compound **1** administered to the patient. The unit dosage form may be, for example, a pill, capsule, or tablet. In an embodiment of the invention, the unit dosage form is a tablet. In an embodiment of the invention, the unit dosage form comprises about 10 mg to about 2000 mg of Compound **1.** In an embodiment of the invention, the unit dosage form comprises about 50 mg or 200 mg of Compound **1** and is in the form of a tablet. In another embodiment of the invention, the unit dosage form comprises about 125 mg or 150 mg of Compound **1** and is in the form of a tablet.

In yet another embodiment of the invention, the purity of Compound **1** is not less than 95%. More preferably, the purity of Compound **1** is not less than 98%.

The concentration of Compound **1** in the bloodstream may be measured as the plasma concentration (ng/mL). Pharmacokinetic parameters for determining the plasma concentration include, but are not limited to, the maximum observed plasma concentration (Cₘₐₓ), area under the plasma concentration time curve (AUC) from time zero up to the last quantifiable time point (AUC₀₋ₜ), AUC from time zero to infinity (AUC_{0-inf}), time of maximum observed plasma concentration after administration (tₘₐₓ), and half-life of Compound **1** in plasma (t_{1/2}).

Administration of Compound **1** with food results in increased bioavailability of Compound **1** as evidenced by an increase in Cₘₐₓ and/or AUC_{0-inf} of Compound **1** as compared to the values if Compound **1** was administered without food.

Administration of Compound 1 with food according to the method of the invention may also increase absorption of Compound **1.** Absorption of Compound **1** may be measured by the concentration attained in the bloodstream over time after administration of Compound **1.** An increase in absorption by administration of Compound **1** with food may also be evidenced by an increase in Cₘₐₓ and/or AUC₀-_{inf}of Compound **1** as compared to the values if Compound **1** was administered without food.

The present invention also provides a method for the treatment or prophylaxis of diseases, disorders, and conditions. An example of a disease, disorder, or condition includes, but is not limited to, a retrovirus infection, or a disease, disorder, or condition associated with a retrovirus infection. Retroviruses are RNA viruses and are generally classified into the alpharetrovirus, betaretrovirus, deltaretrovirus, epsilonretrovirus, gammaretrovirus, lentivirus, and spumavirus families. Examples of retroviruses include, but are not limited to, human immunodeficiency virus (HIV), human T-lymphotropic virus (HTLV), rous sarcoma virus (RSV), and the avian leukosis virus. In general, three genes of the retrovirus genome code for the proteins of the mature virus: *gag* (group-specific antigen) gene, which codes for the core and structural proteins of the virus; *pol* (polymerase) gene, which codes for the enzymes of the virus, including reverse transcriptase, protease, and integrase; and *env* (envelope) gene, which codes for the retrovirus surface proteins.

Retroviruses attach to and invade a host cell by releasing a complex of RNA and the *pol* products, among other things, into the host cell. The reverse transcriptase then produces double stranded DNA from the viral RNA. The double stranded DNA is imported into the nucleus of the host cell and integrated into the host cell genome by the viral integrase. A nascent virus from the integrated DNA is formed when the integrated viral DNA is converted into mRNA by the host cell polymerase and the proteins necessary for virus formation are produced by the action of the virus protease. The virus particle undergoes budding and is released from the host cell to form a mature virus.

One embodiment of the invention provides a method for inhibiting activity of a retrovirus integrase in a patient, comprising administering to the patient a therapeutically effective amount of Compound 1 with food. In a particular embodiment of the invention, the retrovirus is HIV. As used herein, "inhibiting" refers to the decrease or cessation of at least one activity associated with a protein, enzyme, or any other compound.

Another embodiment of the invention provides a method for the treatment or prophylaxis of a retrovirus infection comprising administering to the patient a therapeutically effective amount of Compound 1 with food. In a particular embodiment of the invention, the retrovirus is HIV.

Compound **1** may be administered to a patient in any conventional manner. While it is possible for Compound **1** to be administered as a raw compound, it is preferably administered as a pharmaceutical composition. A "pharmaceutical composition comprising Compound **1**" refers to a pharmaceutical composition comprising Compound **1,** or a pharmaceutically acceptable salt thereof, with one or more pharmaceutically acceptable carriers or excipients and optionally other therapeutic agents and/or components. The salt, carrier, or excipient must be acceptable in the sense of being compatible with the other ingredients and not deleterious to the recipient thereof. Examples of carriers or excipients for oral administration include cornstarch, lactose, magnesium stearate, talc, microcrystalline cellulose, stearic acid, povidone, crospovidone, dibasic calcium phosphate, sodium starch glycolate, hydroxypropyl cellulose (e.g., low substituted hydroxypropyl cellulose), hydroxypropylmethyl cellulose (e.g., hydroxypropylmethyl cellulose 2910), and sodium lauryl sulfate.

The pharmaceutical compositions may be prepared by any suitable method, such as those methods well known in the art of pharmacy, for example, methods such as those described in Gennaro et al., Remington's Pharmaceutical Sciences (18th ed., Mack Publishing Co., 1990), especially Part 8: Pharmaceutical Preparations and their Manufacture. Such methods include the step of bringing into association Compound **1** with the carrier or excipient and optionally one or more accessory ingredients. Such accessory ingredients include those conventional in the art, such as, fillers, binders, diluents, disintegrants, lubricants, colorants, flavoring agents, and wetting agents.

The pharmaceutical compositions may provide controlled, slow release, or sustained release of the Compound **1** over a period of time. The controlled, slow release, or sustained release of Compound **1** may maintain Compound **1** in the bloodstream of the patient for a longer period of time than with conventional formulations. Pharmaceutical compositions include, but are not limited to, coated tablets, pellets, and capsules, and dispersions of Compound **1** in a medium that is insoluble in physiologic fluids or where the release of the therapeutic compound follows degradation of the pharmaceutical composition due to mechanical, chemical, or enzymatic activity.

The pharmaceutical composition of the invention may be, for example, in the form of a pill, capsule, or tablet, each containing a predetermined amount of Compound **1** and preferably coated for ease of swallowing, in the form of a powder or granules, or in the form of a solution or suspension. In an embodiment of the invention, the pharmaceutical composition is in the form of a tablet comprising Compound **1** and the components of the tablet utilized and described in the Examples herein.

For oral administration, fine powders or granules may contain diluting, dispersing, and or surface active agents and may be present, for example, in water or in a syrup, in capsules or sachets in the dry state, or in a nonaqueous solution or suspension wherein suspending agents may be included, or in tablets wherein binders and lubricants may be included. The pharmaceutical composition may also include additional components such as sweeteners, flavoring agents, preservatives (*e.g*., antimicrobial preservatives), suspending agents, thickening agents, and/or emulsifying agents.

When administered in the form of a liquid solution or suspension, the formulation may contain Compound **1** and purified water. Optional components in the liquid solution or suspension include suitable sweeteners, flavoring agents, preservatives (*e.g*., antimicrobial preservatives), buffering agents, solvents, and mixtures thereof. A component of the formulation may serve more than one function. For example, a suitable buffering agent also may act as a flavoring agent as well as a sweetener.

Suitable sweeteners include, for example, saccharin sodium, sucrose, and mannitol. A mixture of two or more sweeteners may be used. The sweetener or mixtures thereof are typically present in an amount of from about 0.001 % to about 70% by weight of the total composition. Suitable flavoring agents may be present in the pharmaceutical composition to provide a cherry flavor, cotton candy flavor, or other suitable flavor to make the pharmaceutical composition easier for a patient to ingest. The flavoring agent or mixtures thereof are typically present in an amount of about 0.0001% to about 5% by weight of the total composition.

Suitable preservatives include, for example, methylparaben, propylparaben, sodium benzoate, and benzalkoniyum chloride. A mixture of two or more preservatives may be used. The preservative or mixtures thereof are typically present in an amount of about 0.0001 % to about 2% by weight of the total composition.

Suitable buffering agents include, for example, citric acid, sodium citrate, phosphoric acid, potassium phosphate, and various other acids and salts. A mixture of two or more buffering agents may be used. The buffering agent or mixtures thereof are typically present in an amount of about 0.001 % to about 4% by weight of the total composition.

Suitable solvents for a liquid solution or suspension include, for example, sorbital, glycerin, propylene glycol, and water. A mixture of two or more solvents may be used. The solvent or solvent system is typically present in an amount of about 1% to about 90% by weight of the total composition.

The pharmaceutical composition may be co-administered with adjuvants. For example, nonionic surfactants such as polyoxyethylene oleyl ether and n-hexadecyl polyethylene ether may be administered with or incorporated into the pharmaceutical composition to artificially increase the permeability of the intestinal walls. Enzymatic inhibitors may also be administered with or incorporated into the pharmaceutical composition.

The present invention also provides a kit comprising (i) a pharmaceutical composition comprising Compound 1, or a pharmaceutically acceptable salt thereof, and a pharmaceutically acceptable carrier, (ii) prescribing information, and (iii) a container. The prescribing information may provide prescribing information conforming to the methods of the invention and/or as otherwise discussed herein. In an embodiment of the invention, the prescribing information includes orally administering Compound 1 with food, to improve the bioavailability and absorption of Compound 1.

Compound 1 may be provided to a patient in a container associated with prescribing information that advises the patient to orally administer Compound 1 with food and may also explain that doing so will increase the bioavailability of Compound 1. Compound 1 may also be provided to a patient in a container associated with prescribing information that advises the patient that the administration of Compound 1 with food results in an increase in absorption of Compound 1, as reflected by an increase in the maximum plasma concentration of Compound 1 as compared to the administration of the compound under fasted conditions.

The effect of food on the pharmacokinetic properties of Compound **1** and upon the therapeutic effectiveness of Compound **1** is illustrated by the following Examples, which are not intended to be limiting in any way.

### Example 6

Unit dosage forms of Compound **1** in 50 mg and 200 mg tablets were prepared for clinical studies of Compound **1.** Compound **1** exists in Form I, Form II, and Form III. Form III was used in the final formulations but Form II or a mixed crystal of Form II and Form III was also used during formulation studies.

Preparation of 200 mg tablets of Compound **1.** The final composition of the 200 mg tablet is shown in Table 1.

**Table 1.**

| **Component** | **Function** | **Amount Per Tablet** |
|---|---|---|
| Compound **1** | Drug substance | 200.0 mg |
| D-mannitol | Diluent | 107.6 mg |
| Light anhydrous silicic acid | Glidant | 25.0 mg |
| Sodium lauryl sulfate | Surfactant | 10.0 mg |
| Crospovidone | Disintegrant | 25.0 mg |
| Hydroxypropylmethylcellulose 2910 (3 mm²/s) | Binder | 20.0 mg |
| Purified water^{*1} | Binder agent | - |
| Croscarmellose sodium | Disintegrant | 100.0 mg |
| Magnesium Stearate | Lubricant | 2.4 mg |
| Total tablet weight | | 490.0 mg |

| | | |
|---|---|---|
| ^{*1} The purified water is removed during processing. | | |

Compound **1** was first micronized with a jet mill. The micronized compound was mixed with D-mannitol (Japanese Pharmacopoeia, JP), crospovidone (Japanese Pharmaceutical Excipients, JPE), and light anhydrous silicic acid (JP) in a polyethylene (PE) bag and then passed though a 500 µm screen three times. Hydroxypropylmethyl-cellulose (HPMC) 2910 (3 mm²/s) (JP) was separately dissolved in purified water by stirring and sodium lauryl sulfate (JP) was added and dissolved. The D-mannitol/crospovidone/light anhydrous silicic acid/Compound **1** mixture was placed in a fluidized-bed granulator and was granulated using the HPMC/sodium lauryl sulfate solution. After granulation, the wet granulates were dried in the same granulator. The dried granules were passed through a 500 µm screen.

The screened granules were then mixed with croscarmellos sodium (JPE) in a PE bag and magnesium stearate (JP) was added to the bag. The granules were compressed into tablets using a rotary tableting machine.

Twenty tablets were packaged in a glass bottle with a desiccant and cushion material (PE sheet), and capped with a polypropylene (PP) screw cap.

Preparation of 50 mg tablets of Compound **1**. The final composition of the 50 mg tablet is shown in Table 2.

**Table 2.**

| **Component** | **Function** | **Amount Per Tablet** |
|---|---|---|
| Compound **1** | Drug substance | 50.0 mg |
| D-mannitol | Diluent | 26.9 mg |
| Light anhydrous silicic acid | Glidant | 6.25 mg |
| Sodium lauryl sulfate | Surfactant | 2.5 mg |
| Crospovidone | Disintegrant | 6.25 mg |
| Hydroxypropylmethylcellulose 2910 (3 mm²/s) | Binder | 5.0 mg |
| Purified water^{*1} | Binder agent | - |
| D-mannitol | Diluent | 145.35 mg |
| Microcrystalline cellulose | Diluent | 145.35 mg |
| Croscarmellose sodium | Disintegrant | 100.0 mg |
| Magnesium Stearate | Lubricant | 2.4 mg |
| Total tablet weight | | 490.0 mg |

| | | |
|---|---|---|
| ^{*1} The purified water is removed during processing. | | |

The size and weight of the 50 mg tablets were the same as the 200 mg tablets. To simplify the manufacturing process, the formulation of the 50 mg tablets was carried out by using the screened granules of Compound **1** prepared as described above as the starting material and diluting the granules with direct compressible excipients. D-mannitol and microcrystalline cellulose were selected as diluting excipients because of compatibility with Compound **1,** and the compressibility and disintegration property of the tablets. Table 3 shows the compositions of the tablets using the screened granules of Compound **1** as starting material.

**Table 3.**

| **Component** | **Quantity Per Tablet** |
|---|---|
| Compound **1** granules | 96.9 mg |
| D-mannitol | 145.35 mg |
| Microcrystalline cellulose | 145.35 mg |
| Croscarmellose sodium | 100.0 mg |
| Magnesium Stearate | 2.4 mg |
| Total tablet weight | 490.0 mg |

The screened granules prepared above were mixed with croscarmellos sodium (JPE), D-mannitol (JP), and microcrystalline cellulose (JP) in a PE bag, followed by addition of magnesium stearate (JP) to the bag. The granules were then compressed into tablets using a rotary tableting machine.

Twenty tablets were packaged in a glass bottle with a desiccant and cushion material (PE sheet), and capped with a polypropylene (PP) screw cap.

### Example 7

The effect of food on the pharmacokinetic properties of Compound 1 was identified in a single blind, randomized, placebo-controlled single oral dose escalation study conducted on 32 Japanese male healthy volunteers.

The following criteria were used to select the subjects for the study:
(1) Healthy Japanese males aged 20 to 35 years;
(2) Body mass index (BMI) of 18.5 to 25.0, wherein BMI =[body weight (kg)/height (m)]²; and
(3) Subjects who gave written informed consent prior to participation in the study.

As part of the larger study, eight subjects (6 active and 2 placebo) received a 400 mg dose in the fasted and fed states. Subjects in the 400 mg fasted cohort received an additional 400 mg dose with breakfast after a washout period (generally a minimum of ten days after the initial administration). The interval of ten days between the two doses was considered appropriate for eliminating any within-subject carryover effects.

Duration of treatment, dose, mode of administration, and product administered for the food effect component of the larger study are shown in Table 4.

**Table 4.**

| **Duration of treatment** | Single dose | |
|---|---|---|
| **Mode of administration** | Fasted, oral administration | Fed, oral administration |
| **Dose (mg)** | 400 | 400 |
| **Product administered** | Compound 1 200mg or placebo tablets | |
| **Number of tablets** | 2 | 2 |

The subjects were admitted to the medical institution in the afternoon on the day before administration ("Day -1 "). Compound **1** or placebo was administered on the day after hospitalization ("Day 1"). The subjects were discharged the day after administration ("Day 2"). Thus, the subjects were hospitalized for a total of three days. Follow up assessment for safety was performed 6 to 8 days after administration ("Days 7 to 9").

Compound **1** or placebo was administered orally with 200 mL water. Doses were administered at similar times for each subject in each treatment period. All subjects fasted from food and fluids from the time after dinner on the day prior to dosing (Day -1) until breakfast on Day 1 (for subjects receiving Compound **1** or placebo in the fed state) or lunch-time on Day 1 (for subjects in the fasted state).

When subjects were administered with Compound **1** in the fed state, they received a breakfast about 30 minutes prior to dosing. The breakfast consisted of the following:
160 mL apple juice
Boiled egg (50 g)
Bread roll (105 g)
8 g butter
14 g strawberry jam
1 Total energy content: 574.6 Kcal; Total fat content: 21.4 g (33% of total calories); Total protein: 17.5 g (12% total calories); Total carbohydrate: 79.0 g (55% of total calories).

Blood samples for pharmacokinetic analysis were taken 1.5 hours prior to dosing and at the following times after dosing: 0.25, 0.5, 1, 2, 3, 4, 6, 8, 12, and 24 hours post-dose.

The following pharmacokinetic parameters were calculated for Compound **1** in each study group (fed and fasted conditions):
- tₘₐₓ: Time of maximum observed plasma concentration;
- Cₘₐₓ: Maximum observed plasma concentration;
- t_{1/2 λz}: Elimination half-life in the terminal phase;
- AUC_{0-tz}: Area under the plasma concentration-time curve (AUC) from time zero up to the last quantifiable concentration (AUC (0 - tz)); and
- AUC_{0-inf}: AUC from time zero to infinity.

The plasma concentrations of Compound **1** were determined by the following assay. Plasma samples were isolated from healthy subjects administered with Compound **1.** The plasma samples were extracted by solid phase extraction using Empore^{™} Disk Plate (C8SD), and then measured by high-performance liquid chromatography/tandem mass spectrometry (LC/MS/MS) (HPLC model 2795 separations module from Waters; mass spectrometer model API4000 from MDS SCIEX). Ionization and detection were performed with electrospray ionization and positive ion detection with multiple reaction monitoring, respectively. This analytical method has been validated with respect to: selectivity, linearity, lower limit of quantification (LLOQ), intra- and inter-assay precision and accuracy, standard solution stability, matrix stability, post-preparative stability, recovery and dilution integrity, and considered to be sufficiently reliable as a method for determination of Compound **1** over the concentration range of 1 to 1000 ng/mL when 50 µL of human plasma were used.

To 50µL of plasma samples spiked with 10 mL each of 90% acetonitrile and 20 mmol/L ammonium formate-formic acid buffer (pH 3.0)/acetonitrile (10:90, v/v), 10 µL each of internal standard solution was added. To these samples, 200 µL of 0.1 % formic acid in 10% acetonitrile was added. These solutions were then mixed for 10 seconds and centrifuged at 10,000 rpm for 5 minutes at 4°C. A 250 µL aliquot of the supernatant was added to a Empore^{™} Disk Plate (C8SD) (conditioned with 150 µL of acetonitrile and 200 µL of 0.1 % formic acid in this order). The plate was washed twice with 200 µL of 0.1 % formic acid in 20% acetonitrile and eluted twice with 100 µL of 0.1% formic acid in 80% acetonitrile (200 µL in total). To the eluates, 200 µL of 0.1 % formic acid was added and mixed. Then, 10 µL of each solution was injected into the LC/MS/MS system and analyzed.

Geometric means ratio (fed/fasted) with 90% confidence intervals were estimated from Cₘₐₓ and AUC_{0-1nf} following oral administration of 400 mg of Compound **1** in the fasted and fed states.

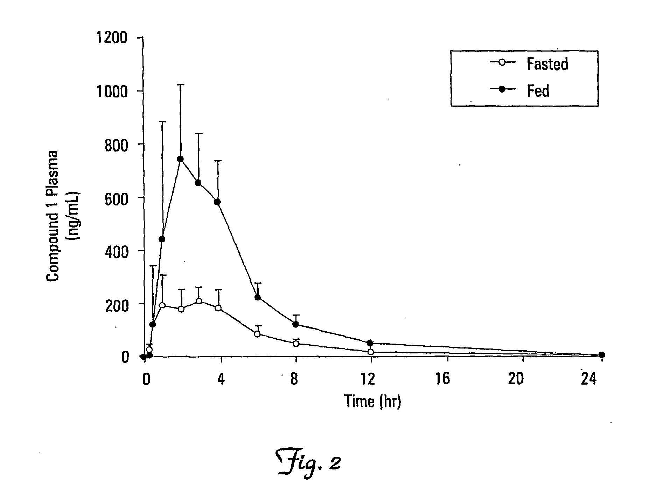

The mean plasma concentration over time following oral administration of 400 mg of Compound **1** in the fasted and fed states are shown in Figure 2. The mean test results of the pharmacokinetic parameters, AUC_{0-1nf} (ng·hr/mL), Cₘₐₓ (ng/mL), t_{1/2 λz} (hr), and tₘₐₓ (hr) of Compound **1** in the fasted and fed states are summarized in Table 5, and are tabulated in Table 6.

**Table 5.**

| | **Administration condition** | **tₘₐₓ (hr)** | **Cₘₐₓ** (ng/mL) | **t_{1/2 λz} (hr)** | **AUC_{0-inf} (ng·-hr/mL)** |
|---|---|---|---|---|---|
| Compound **1** | Fasted | 2.5 ± 1.2 | 264 ± 78 | 5.4 ± 1.0 | 1451 ± 308 |
| | Fed | 2.3 ± 1.0 | 903 ± 391 | 3.2 ± 0.3 | 3942 ± 1072 |

| | | | | | |
|---|---|---|---|---|---|
| Dose: 400 mg Mean ± SD (n=6) | | | | | |

**Table 6.**

| | **Pharmacokinetic parameter** | **Fed/Fasted** | | |
|---|---|---|---|---|
| | | **Geometric means ratio** | **90% Confidence interval** | |
| | | | **Lower limit** | **Upper limit** |
| Compound **1** | Cₘₐₓ | 3.30 | 2.27 | 4.80 |
| | AUC_{0-1nf} | 2.69 | 2.16 | 3.36 |

| | | | | |
|---|---|---|---|---|
| Dose: 400 mg n=6 subjects/each group | | | | |

Figure 2, Table 5, and Table 6 show that when Compound **1** was administered in the fed state, Cₘₐₓ and AUC_{0-1nf} of the unchanged drug were 3.30 and 2.69-fold higher, respectively, compared to those in the fasted state. These results demonstrate that bioavailability and absorption of Compound **1** increased in the presence of food.

The observed increases in the pharmacokinetic parameters when Compound 1 was administered with food indicate that Compound **1** is more readily absorbed when administered with food, such as after a meal. Thus, the administration of Compound **1** with food results in an increase in the bioavailability of the drug when compared to the administration of the drug under fasted conditions. In addition, Compound **1** was safe and well tolerated with no serious adverse events. All adverse events were mild. No clinically significant electrocardiogram (ECG) changes were noted.

All references, including publications, patent applications, and patents, cited herein are hereby incorporated by reference in their entirety.

The use of the terms "a" and "an" and "the" and similar referents in the context of describing the invention (including the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein may be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The embodiments within the specification provide an illustration of embodiments of the invention and should not be construed to limit the scope of the invention. The skilled artisan recognizes that many other embodiments are encompassed by the claimed invention and that it is intended that the specification and examples be considered as exemplary only, with the true scope and spirit of the invention being indicated by the following claims.

Embodiments of the invention:
1. The use of ritonavir or a pharmaceutically acceptable salt thereof for the manufacture of a medicament for improving the pharmacokinetics of an HIV integrase inhibitor or a pharmaceutically acceptable salt thereof in a patient.
2. The use of embodiment 1 wherein the integrase inhibitor is a compound of formula (I) or a pharmaceutically acceptable salt thereof: 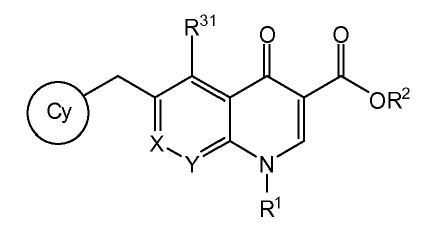 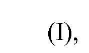 where,
   ring Cy is a C₃₋₁₀ carbon ring group or a heterocyclic group, each group being optionally substituted by 1 to 5 substituents selected from group A;
   the heterocyclic group is a saturated or unsaturated ring comprising at least one heteroatom selected from the group consisting of nitrogen, oxygen and sulfur;
   group A is cyano, phenyl, nitro, halogen, C₁₋₄ alkyl, halo C₁₋₄ alkyl, halo C₁₋₄ alkyloxy, -OR^{a1}, -SR^{a1}, -NR^{a1}R^{a2}, -CONR^{a1}R^{a2}, -SO₂NR^{a1}R^{a2}, -COR^{a3}, - NR^{a1}COR^{a3}, -SO₂R^{a3}, -NR^{a1}SO₂R^{a3}, -COOR^{a1} or -NR^{a2} COOR^{a3};
   R^{a1} and R^{a2} are the same or different and each is H, C₁₋₄ alkyl or benzyl;
   R^{a3} is C₁₋₄ alkyl;
   R¹ is selected from group B or is C₁₋₁₀ alkyl optionally substituted by 1 to 3 substituents selected from halogen or group B;
   group B is:
   a C₃₋₁₀ carbon ring optionally substituted by 1 to 5 substituents selected from group A,
   a heterocyclic group optionally substituted by 1 to 5 substituents selected from group A,
      - OR^{a4},
      - SR^{a4}
      - NR^{a4}R^{a5},
      - CONR^{a4}R^{a5},
      - SO₂NR^{a4}R^{a5},
      - COR^{a6},
      - NR^{a4}COR^{a6},
      - SO₂R^{a6},
      - NR^{a4}SO₂R^{a6},
      - COOR^{a4} or
      - NR^{a5} COOR^{a6};
   R^{a4} and R^{a5} are the same or different and each is:
   H,
   C₁₋₄ alkyl,
   a C₃₋₁₀ carbon ring group optionally substituted by 1 to 5 substituents selected from group A or
   a heterocyclic group optionally substituted by 1 to 5 substituents selected from the group A; R^{a6} is
   C₁₋₄ alkyl,
   a C₃₋₁₀ carbon ring group optionally substituted by 1 to 5 substituents selected from group A or
   a heterocyclic group optionally substituted by 1 to 5 substituents selected from group A;
   R² is H or C₁₋₄ alkyl;
   R³¹ is H, cyano, hydroxy, amino, nitro, halogen, C₁₋₄ alkyl, C₁₋₄ alkoxy, C₁₋₄ alkylsulfanyl, halo C₁₋₄ alkyl or halo C₁₋₄ alkyloxy group;
   X is C-R³² or N;
   Y is C-R³³ or N;
   R³² and R³³ are the same or different and each is:
      H,
      cyano,
      nitro,
      halogen,
      a C₃₋₁₀ carbon ring group optionally substituted by 1 to 5 substituents selected from group A,
      a heterocyclic group optionally substituted by 1 to 5 substituents selected from group A, C₁₋₁₀ alkyl optionally substituted by 1 to 3 substituents selected from halogen or group B,
         - OR^{a7},
         - SR^{a7},
         - NR^{a7}R^{a8},
         - NR^{a7}COR^{a9},
         - COOR^{a10} or
         - N=CH-NR^{a10}R^{a11};
         R^{a7} and R^{a8} are the same or different and each is selected from H, group B or C₁₋₁₀ alkyl optionally substituted by 1 to 3 substituents selected from halogen or group B;
         R^{a9} is C₁₋₄ alkyl; and
         R^{a10} and R^{a11} are the same or different and each is H or C₁₋₄ alkyl;
         in a patient.
3. The use of embodiment 1 wherein the blood level of the integrase inhibitor or the pharmaceutically acceptable salt thereof is increased.
4. The use of ritonavir or a pharmaceutically acceptable salt thereof, and a compound of Formula (I): 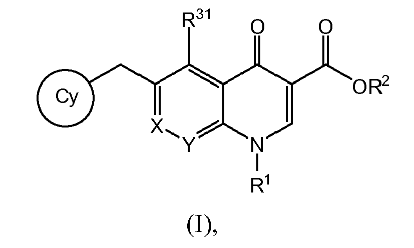 or a pharmaceutically acceptable salt thereof,
   where,
   ring Cy is a C₃₋₁₀ carbon ring group or a heterocyclic group, each group being optionally substituted by 1 to 5 substituents selected from group A;
   the heterocyclic group is a saturated or unsaturated ring comprising at least one heteroatom selected from the group consisting of nitrogen, oxygen and sulfur;
   group A is cyano, phenyl, nitro, halogen, C₁₋₄ alkyl, halo C₁₋₄ alkyl, halo C₁₋₄ alkyloxy, -OR^{a1} -SR^{a1}, -NR^{a1}R^{a2}, -CONR^{a1}R^{a2}, -SO₂NR^{a1}R^{a2}, -COR^{a3},-NR^{a1}COR^{a3}, -SO₂R^{a3}, -NR^{a1}SO₂R^{a3}, -COOR^{a1} or -NR^{a2} COOR^{a3};
   R^{a1} and R^{a2} are the same or different and each is H, C₁₋₄ alkyl or benzyl;
   R^{a3} is C₁₋₄ alkyl;
   R¹ is selected from group B or is C₁₋₁₀ alkyl optionally substituted by 1 to 3 substituents selected from halogen or group B;
   group B is:
   a C₃₋₁₀ carbon ring optionally substituted by 1 to 5 substituents selected from group A,
   a heterocyclic group optionally substituted by 1 to 5 substituents selected from group A,
      - OR^{a4},
      - SR^{a4},
      - NR^{a4}R^{a5},
      - CONR^{a4}R^{a5},
      - SO₂NR^{a4}R^{a5},
      - COR^{a6},
      - NR^{a4}COR^{a6},
      - SO₂R^{a6},
      - NR^{a4}SO₂R^{a6},
      - COOR^{a4} or
      - NR^{a5} COOR^{a6};
   R^{a4} and R^{a5} are the same or different and each is:
   H,
   C₁₋₄ alkyl,
   a C₃₋₁₀ carbon ring group optionally substituted by 1 to 5 substituents selected from group A or
   a heterocyclic group optionally substituted by 1 to 5 substituents selected from the group A;
   R^{a6} is
   C₁₋₄ alkyl,
   a C₃₋₁₀ carbon ring group optionally substituted by 1 to 5 substituents selected from group A or
   a heterocyclic group optionally substituted by 1 to 5 substituents selected from group A;
   R² is H or C₁₋₄ alkyl;
   R³¹ is H, cyano, hydroxy, amino, nitro, halogen, C₁₋₄ alkyl, C₁₋₄ alkoxy, C₁₋₄ alkylsulfanyl, halo C₁₋₄ alkyl or halo C₁₋₄ alkyloxy group;
   X is C-R³² or N;
   Y is C-R³³ or N;
   R³² and R³³ are the same or different and each is:
      H,
      cyano,
      nitro,
      halogen,
      a C₃₋₁₀ carbon ring group optionally substituted by 1 to 5 substituents selected from group A,
      a heterocyclic group optionally substituted by 1 to 5 substituents selected from group A, C₁₋₁₀ alkyl optionally substituted by 1 to 3 substituents selected from halogen or group B,
         - OR^{a7},
         - SR^{a7},
         - NR^{a7}R^{a8},
         - NR^{a7}COR^{a9},
         - COOR^{a10} or
         - N=CH-NR^{a10}R^{a11},
         R^{a7} and R^{a8} are the same or different and each is selected from H, group B or C₁₋₁₀ alkyl optionally substituted by 1 to 3 substituents selected from halogen or group B;
         R^{a9} is C₁₋₄ alkyl; and
         R^{a10} and R^{a11} are the same or different and each is H or C₁₋₄ alkyl;
         for the manufacture of a medicament for inhibiting HIV integrase in a patient.
5. The use of embodiment 4 wherein the compound of Formula (I) or the pharmaceutically acceptable salt thereof is an HIV integrase inhibitor.
6. The use of any one of embodiments 2-5 wherein the compound of Formula (I) is 6-(3-chloro-2-fluorobenzyl)-1-[(*2S*)-1-hydroxy-3-methylbutan-2-yl]-7-methoxy-4-oxo-1,4-dihydroquinoline-3-carboxylic acid.
7. The use of any one of embodiments 1-6, wherein the compound or its pharmaceutically acceptable salt and ritonavir or its pharmaceutically acceptable salt are administered to the patient as a single composition to the patient.
8. The use of any one of embodiments 1-7 wherein the compound or its pharmaceutically acceptable salt and ritonavir or its pharmaceutically acceptable salt are administered orally.
9. The use of embodiment 8, wherein the oral administration is once a day.
10. The use of any one of embodiments 1-9 wherein the patient is also receiving one or more agents selected the group consisting of stavudine, emtricitabine, tenofovir, emtricitabine, abacavir, lamivudine, zidovudine, didanosine, zalcitabine, phosphazide, efavirenz, nevirapine, delavirdine, tipranavir, saquinavir, indinavir, atazanavir, nelfinavir, amprenavir, samprenavir, , fosamprenavir, lopinavir, ritonavir, enfuvirtide, Fozivudine tidoxil, Alovudine, Dexelvucitabine, Apricitabine, Amdoxovir, Elvucitabine (ACH126443), Racivir (racemic FTC, PSI-5004), MIV-210, KP-1461, fosalvudine tidoxil (HDP 99.0003), AVX756, Dioxolane Thymine (DOT), TMC-254072, INK-20, 4'-Ed4T, TMC-125 (etravirine), Capravirine, TMC-278 (rilpivirine), GW-695634, Calanolide A, BILR 355 BS, and VRX 840773, and pharmaceutically acceptable salts thereof.
11. Use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or its pharmaceutically acceptable salt for the manufacture of a medicament for increasing the bioavailability of the compound comprising administering to a patient a therapeutically effective amount of the compound or a pharmaceutically acceptable salt thereof to be administered with food.
12. Use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or its pharmaceutically acceptable salt for the manufacture of a medicament for increasing the absorption of the compound in a patient, comprising administering to the patient a therapeutically effective amount of the compound or a pharmaceutically acceptable salt thereof to be administered with food.
13. The use of embodiment 11 or 12, wherein said administering results in a maximum plasma concentration of the compound greater than if the compound or the pharmaceutically acceptable salt thereof was administered without food.
14. The use of embodiment 11 or 12, wherein said administering results in an area under the plasma concentration time curve from time zero to infinity (AUC₀-inf) of the compound greater than if the compound or the pharmaceutically acceptable salt thereof was administered without food.
15. Use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or its pharmaceutically acceptable salt for the manufacture of a medicament for inhibiting activity of a retrovirus integrase in a patient, comprising administering to the patient a therapeutically effective amount of the compound or a pharmaceutically acceptable salt thereof to be administered with food.
16. Use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or its pharmaceutically acceptable salt for the manufacture of a medicament for the treatment or prophylaxis of a retrovirus infection in a patient, comprising administering to the patient a therapeutically effective amount of the compound or a pharmaceutically acceptable salt thereof to be administered with food.
17. The use of embodiment 15 or 16, wherein the retrovirus is human immunodeficiency virus (HIV).
18. The use of any one of embodiments 11-117 wherein the therapeutically effective amount is about 10 mg to about 2000 mg.
19. The use of any one of embodiments 11-19, wherein the compound or the pharmaceutically acceptable salt thereof is administered between about one hour prior to the consumption of food to about two hours after the consumption of food.
20. The use of embodiments 19 wherein the compound or the pharmaceutically acceptable salt thereof is administered substantially at the same time as the consumption of food.
21. The use of embodiments 19 wherein the compound or the pharmaceutically acceptable salt thereof is administered immediately after the consumption of food and up to about one hour after consumption of the food.
22. The use of any one of embodiments 11-21 wherein the compound or the pharmaceutically acceptable salt thereof is administered in a pharmaceutical composition.
23. The use of embodiment 22, wherein the pharmaceutical composition is in a unit dosage form of a tablet.
24. The use of any one of embodiments 11-23 wherein the compound or the pharmaceutically acceptable salt thereof is administered orally.
25. A kit comprising: (1) a pharmaceutical composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, and a pharmaceutically acceptable carrier; (2) prescribing information; and (3) a container; wherein the prescribing information includes advice regarding administering 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof with food.
26. The kit of embodiment 25, wherein the prescribing information includes a description of increased bioavailability of 6-(3-chloro-2-fluorobenzyl)-1-[(*2S*)-1-hydroxy-3-methylbutan-2-yl]-7-methoxy-4-oxo-1,4-dihydroquinoline-3-carboxylic acid when administered with food than without food.
27. The kit of embodiment 25, wherein the prescribing information includes advice regarding administering 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof between about one hour prior to the consumption of food to about two hours after the consumption of food.
28. The kit of embodiment 25, wherein the prescribing information includes advice regarding administering 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof substantially at the same time as the consumption of food.
29. The kit of embodiment 25, wherein the prescribing information includes advice regarding administering 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof immediately after the consumption of food and up to about one hour after the consumption of food.
30. The kit of any one of embodiments 25-29 wherein the pharmaceutical composition is in a unit dosage form of a tablet.
31. The use of any one of embodiments 1-10 wherein the medicament is for administration to the patient with food.
32. The use of any one of embodiments 1-10 wherein the medicament is formulated as a unit dosage form tablet.
33. The kit of any one of embodiments 25-29 further comprising ritonavir, or a pharmaceutically acceptable salt thereof.
34. Use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for the manufacture of a medicament for increasing the bioavailability of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid in a patient when the medicament is administered with food, to be administered with ritonavir or a pharmaceutically acceptable salt thereof.
35. Use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for the manufacture of a medicament for increasing the absorption of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid in a patient when the medicament is administered with food, to be administered with ritonavir or a pharmaceutically acceptable salt thereof.
36. Use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for the manufacture of a medicament for inhibiting activity of a retrovirus integrase in a patient when the medicament is administered with food, to be administered with ritonavir or a pharmaceutically acceptable salt thereof.
37. Use of the compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for the manufacture of a medicament for the treatment or prophylaxis of a retrovirus infection in a patient when the medicament is administered with food, to be administered with ritonavir or a pharmaceutically acceptable salt thereof.
38. Pharmaceutical composition comprising ritonavir or a pharmaceutically acceptable salt thereof for improving the pharmacokinetics of an HIV integrase inhibitor in a patient.
39. The pharmaceutical composition of embodiment 38 to be administered with food.
40. A pharmaceutical composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for increasing the bioavailability of the compound to be administered with food.
41. A pharmaceutical composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for increasing the absorption of the compound in a patient to be administered with food.
42. A pharmaceutical composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for inhibiting activity of a retrovirus integrase in a patient to be administered with food.
43. A pharmaceutical composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for the treatment or prophylaxis of a retrovirus infection in a patient to be administered with food.
44. The pharmaceutical composition of any of embodiment 40-43 to be administered with ritonavir or a pharmaceutically acceptable salt thereof.
45. An anti-retroviral agent comprising ritonavir or a pharmaceutically acceptable salt thereof for improving the pharmacokinetics of an HIV integrase inhibitor in a patient.
46. The anti-retroviral agent composition of embodiment 45 to be administered with food.
47. An anti-retroviral agent composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for increasing the bioavailability of the compound to be administered with food.
48. An anti-retroviral agent comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof for increasing the absorption of the compound in a patient to be administered with food.
49. An anti-retroviral agent comprising 6-(3-chloro-2-fluorobenzyl)-1-[(2S)-1-hydroxy-3-methylbutan-2-yl]-7-methoxy-4-oxo-1,4-dihydroquinoline-3-carboxylic acid or a pharmaceutically acceptable salt thereof for inhibiting activity of a retrovirus integrase in a patient to be administered with food.
50. An anti-retroviral agent comprising 6-(3-chloro-2-fluorobenzyl)-1-[(2S)-1-hydroxy-3-methylbutan-2-yl]-7-methoxy-4-oxo-1,4-dihydroquinoline-3-carboxylic acid or a pharmaceutically acceptable salt thereof for the treatment or prophylaxis of a retrovirus infection in a patient to be administered with food.
51. The anti-retroviral agent composition of any of embodiment 47-50 to be administered with ritonavir or a pharmaceutically acceptable salt thereof.

## Claims

1. The compound (2S,3S,5S)-5-(N-(N-((N-methyl-N-((2-isopropyl-4-thiazolyl)methyl)amino)-carbonyl)-L-valinyl)amino)-2-(N-((5-thiazolyl)methoxycarbonyl)amino)-1,6-diphenyl-3-hydroxyhexane (ritonavir) or a pharmaceutically acceptable salt thereof for use in the form of a medicament for improving the pharmacokinetics of an HIV integrase inhibitor or a pharmaceutically acceptable salt thereof in a patient.

2. The compound for use according to claim 1 wherein the integrase inhibitor is a compound of formula (I) or a pharmaceutically acceptable salt thereof: 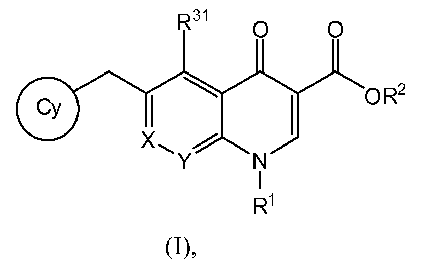 where,
ring Cy is a C₃₋₁₀carbon ring group or a heterocyclic group, each group being optionally substituted by 1 to 5 substituents selected from group A;
the heterocyclic group is a saturated or unsaturated ring comprising at least one heteroatom selected from the group consisting of nitrogen, oxygen and sulfur;
group A is cyano, phenyl, nitro, halogen, C₁₋₄ alkyl, halo C₁₋₄ alkyl, halo C₁₋₄ alkyloxy, -OR^{a1}, -SR^{a1}, -NR^{a1}R^{a2}, -CONR^{a1}R^{a2}, -SO₂NR^{a1}R^{a2}, -COR^{a3},-NR^{a1}COR^{a3}, -SO₂R^{a3}, -NR^{a1}SO₂R^{a3}, -COOR^{a1} or -NR^{a2} COOR^{a3};
R^{a1} and R^{a2} are the same or different and each is H, C₁₋₄ alkyl or benzyl;
R^{a3} is C₁₋₄ alkyl;
R¹ is selected from group B or is C₁₋₁₀ alkyl optionally substituted by 1 to 3 substituents selected from halogen or group B;
group B is:
a C₃₋₁₀ carbon ring optionally substituted by 1 to 5 substituents selected from group A,
a heterocyclic group optionally substituted by 1 to 5 substituents selected from group A,
- OR^{a4},
- SR^{a4},
- NR^{a4}R^{a5},
- CONR^{a4}R^{a5},
- SO₂NR^{a4}R^{a5},
- COR^{a6},
- NR^{a4}COR^{a6},
- SO₂R^{a6},
- NR^{a4}SO₂R^{a6},
- COOR^{a4} or
- NR^{a5} COOR^{a6};
R^{a4} and R^{a5} are the same or different and each is:
H,
C₁₋₄ alkyl,
a C₃₋₁₀ carbon ring group optionally substituted by 1 to 5 substituents selected from group A or
a heterocyclic group optionally substituted by 1 to 5 substituents selected from the group A;
R^{a6} is
C₁₋₄ alkyl,
a C₃₋₁₀ carbon ring group optionally substituted by 1 to 5 substituents selected from group A or
a heterocyclic group optionally substituted by 1 to 5 substituents selected from group A;
R² is H or C₁₋₄ alkyl;
R³¹ is H, cyano, hydroxy, amino, nitro, halogen, C₁₋₄ alkyl, C₁₋₄ alkoxy, C₁₋₄ alkylsulfanyl, halo C₁₋₄ alkyl or halo C₁₋₄ alkyloxy group;
X is C-R³² or N;
Y is C-R³³ or N;
R³² and R³³ are the same or different and each is:
H,
cyano,
nitro,
halogen,
a C₃₋₁₀ carbon ring group optionally substituted by 1 to 5 substituents selected from group A,
a heterocyclic group optionally substituted by 1 to 5 substituents selected from group A, C₁₋₁₀ alkyl optionally substituted by 1 to 3 substituents selected from halogen or group B,
- OR^{a7},
- SR^{a7},
- NR^{a7}R^{a8},
- NR^{a7}COR^{a9},
- COOR^{a10} or
- N=CH-NR^{a10}R^{a11};
R^{a7} and R^{a8} are the same or different and each is selected from H, group B or C₁₋₁₀ alkyl optionally substituted by 1 to 3 substituents selected from halogen or group B;
R^{a9} is C₁₋₄ alkyl; and
R^{a10} and R^{a11} are the same or different and each is H or C₁₋₄ alkyl;
in a patient.

3. The compound for use according to claim 1 or 2, wherein the blood level of the integrase inhibitor or the pharmaceutically acceptable salt thereof is increased.

4. The compound (2S,3S,5S)-5-(N-(N-((N-methyl-N-((2-isopropyl-4-thiazolyl)methyl)amino)-carbonyl)-L-valinyl)amino)-2-(N-((5-thiazolyl)methoxycarbonyl)amino)-1,6-diphenyl-3-hydroxyhexane (ritonavir) or a pharmaceutically acceptable salt thereof and a compound of Formula (I): 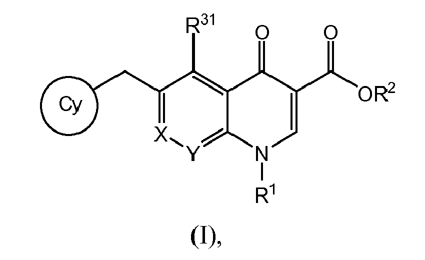 or a pharmaceutically acceptable salt thereof,
where,
ring Cy is a C₃₋₁₀ carbon ring group or a heterocyclic group, each group being optionally substituted by 1 to 5 substituents selected from group A;
the heterocyclic group is a saturated or unsaturated ring comprising at least one heteroatom selected from the group consisting of nitrogen, oxygen and sulfur;
group A is cyano, phenyl, nitro, halogen, C₁₋₄ alkyl, halo C₁₋₄ alkyl, halo C₁₋₄ alkyloxy, -OR^{a1}, -SR^{a1}, -NR^{a1}R^{a2}, -CONR^{a1}R^{a2}, -SO₂NR^{a1}R^{a2}, -COR^{a}3,-NR^{a1}COR^{a3}, -SO₂R^{a3}, -NR^{a1}SO₂R^{a3}, -COOR^{a1} or -NR^{a2} COOR^{a3};
R^{a1} and R^{a2} are the same or different and each is H, C₁₋₄ alkyl or benzyl;
R^{a3} is C₁₋₄ alkyl;
R¹ is selected from group B or is C₁₋₁₀ alkyl optionally substituted by 1 to 3 substituents selected from halogen or group B;
group B is:
a C₃₋₁₀ carbon ring optionally substituted by 1 to 5 substituents selected from group A,
a heterocyclic group optionally substituted by 1 to 5 substituents selected from group A,
- OR^{a4},
- SR^{a4},
- NR^{a4}R^{a5},
- CONR^{a4}R^{a5},
- SO₂NR^{a4}R^{a5},
- COR^{a6},
- NR^{a4}COR^{a6},
- SO₂R^{a6},
- NR^{a4}SO₂R^{a6},
- COOR^{a4} or
- NR^{a5} COOR^{a6};
R^{a4} and R^{a5} are the same or different and each is:
H,
C₁₋₄ alkyl,
a C₃₋₁₀ carbon ring group optionally substituted by 1 to 5 substituents selected from group A or
a heterocyclic group optionally substituted by 1 to 5 substituents selected from the group A;
R^{a6} is
C₁₋₄ alkyl,
a C₃₋₁₀ carbon ring group optionally substituted by 1 to 5 substituents selected from group A or
a heterocyclic group optionally substituted by 1 to 5 substituents selected from group A;
R² is H or C₁₋₄ alkyl;
R³¹ is H, cyano, hydroxy, amino, nitro, halogen, C₁₋₄ alkyl, C₁₋₄ alkoxy, C₁₋₄ alkylsulfanyl, halo C₁₋₄ alkyl or halo C₁₋₄ alkyloxy group;
X is C-R³² or N;
Y is C-R³³ or N;
R³² and R³³ are the same or different and each is:
H,
cyano,
nitro,
halogen,
a C₃₋₁₀ carbon ring group optionally substituted by 1 to 5 substituents selected from group A,
a heterocyclic group optionally substituted by 1 to 5 substituents selected from group A, C₁₋₁₀ alkyl optionally substituted by 1 to 3 substituents selected from halogen or group B,
- OR^{a7},
- SR^{a7},
- NR^{a7}R^{a8},
- NR^{a7}COR^{a9},
- COOR^{a10} or
- N=CH-NR^{a10}R^{a11};
R^{a7} and R^{a8} are the same or different and each is selected from H, group B or C₁₋₁₀ alkyl optionally substituted by 1 to 3 substituents selected from halogen or group B;
R^{a9} is C₁₋₄ alkyl; and
R^{a10} and R^{a11} are the same or different and each is H or C₁₋₄ alkyl;
for use in the form of a medicament for inhibiting HIV integrase in a patient.

5. The compound for use according to any one of claims 2-4wherein the compound of Formula (I) is 6-(3-chloro-2-fluorobenzyl)-1-[(*2S*)-1-hydroxy-3-methylbutan-2-yl]-7-methoxy-4-oxo-1,4-dihydroquinoline-3-carboxylic acid.

6. The compound for use according to any one of claims 1-5 wherein the compound or its pharmaceutically acceptable salt and ritonavir or its pharmaceutically acceptable salt are administered orally.

7. The compound for use according to claim 6, wherein the oral administration is once a day.

8. The compound for use according to any one of claims 1-7 wherein the patient is also receiving one or more agents selected the group consisting of stavudine, emtricitabine, tenofovir, emtricitabine, abacavir, lamivudine, zidovudine, didanosine, zalcitabine, phosphazide, efavirenz, nevirapine, delavirdine, tipranavir, saquinavir, indinavir, atazanavir, nelfinavir, amprenavir, samprenavir, , fosamprenavir, lopinavir, ritonavir, enfuvirtide, Fozivudine tidoxil, Alovudine, Dexelvucitabine, Apricitabine, Amdoxovir, Elvucitabine (ACH126443), Racivir (racemic FTC, PSI-5004), MIV-210, KP-1461, fosalvudine tidoxil (HDP 99.0003), AVX756, Dioxolane Thymine (DOT), TMC-254072, INK-20, 4'-Ed4T, TMC-125 (etravirine), Capravirine, TMC-278 (rilpivirine), GW-695634, Calanolide A, BILR 355 BS, and VRX 840773, and pharmaceutically acceptable salts thereof.

9. The compound for use according to any one of claims 1-8, wherein the medicament is for administration to the patient with food.

10. The compound for use according to any one of claims 1-8, wherein the medicament is formulated as a unit dosage form tablet.

11. The compound 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or its pharmaceutically acceptable salt for use in increasing the bioavailability of the compound, for increasing the absorption of the compound, for inhibiting activity of a retrovirus integrase in a patient, or for the treatment or prophylaxis of a retrovirus infection in a patient wherein the compound or a pharmaceutically acceptable salt thereof is to be administered with food.

12. The compound for use according to claim 11, wherein the compound or the pharmaceutically acceptable salt thereof is administered between about one hour prior to the consumption of food to about two hours after the consumption of food.

13. The compound for use according to claim 11 or 12, wherein the compound or the pharmaceutically acceptable salt thereof is administered orally.

14. A kit comprising: (1) a pharmaceutical composition comprising 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, and a pharmaceutically acceptable carrier; (2) prescribing information; and (3) a container; wherein the prescribing information includes advice regarding administering 6-(3-chloro-2-fluorobenzyl)-1-[(*2S*)-1-hydroxy-3-methylbutan-2-yl]-7-methoxy-4-oxo-1,4-dihydroquinoline-3-carboxylic acid or a pharmaceutically acceptable salt thereof with food.

15. The kit of claim 14 wherein the pharmaceutical composition is in a unit dosage form of a tablet.

16. The kit of claim 14 or 15 further comprising ritonavir, or a pharmaceutically acceptable salt thereof.
